# EUROPÄISCHE PATENTSCHRIFT

(11) **EP 2 391 597 B1**
(45) Veröffentlichungstag und Bekanntmachung des Hinweises auf die Patenterteilung: **22.08.2018**
(21) Anmeldenummer: 10701244.5
(22) Anmeldetag: 27.01.2010
(51) Int. Cl.: C07C 45/28, C07C 45/62, C07C 45/82, C07C 49/607, C07C 49/413

(54) **VERFAHREN ZUR HERSTELLUNG VON REINEM CYCLODODECANON**
METHOD FOR PRODUCING PURE CYCLODODECANONE
PROCÉDÉ DE PRÉPARATION DE CYCLODODÉCANONE PURE

(30) Priorität: 28.01.2009 EP 09151525
(43) Veröffentlichungstag der Anmeldung: 07.12.2011
(73) Patentinhaber: BASF SE, 67056 Ludwigshafen am Rhein (DE)
(72) Erfinder: TELES, Joaquim, Henrique, 67166 Otterstadt (DE); ROESSLER-FEIGEL, Beatrice, 67256 Weisenheim am Sand (DE); HAUK, Alexander, 67076 Ludwigshafen (DE); MEIER, Anton, 67134 Birkenheide (DE); MUELLER, Christian, 68167 Mannheim (DE); SCHELPER, Michael, 67065 Ludwigshafen (DE); KIRCHNER, Tanja, 55283 Nierstein (DE); SZESCHKUS, Susanne, 55232 Alzey (DE); PINKOS, Rolf, 67098 Bad Duerkheim (DE); TEBBEN, Gerd-Dieter, 68239 Mannheim (DE)
(86) Internationale Anmeldenummer: PCT/EP2010/050888
(87) Internationale Veröffentlichungsnummer: WO 2010/086314

(56) Entgegenhaltungen:
- WO-A1-2008/000757
- WO-A2-2005/030690
- DE-A1-102005 048 250

## Beschreibung

Die vorliegende Erfindung betrifft ein Verfahren zur Herstellung mindestens einer cyclischen Verbindung mit Z Cyclen und 7 bis 16 C-Atomen mit einer Ketogruppe, mindestens umfassend die Stufen (a1) Oxidation einer Zusammensetzung (A), mindestens enthaltend ein cyclisches Olefin mit Z Cyclen und 7 bis 16 C-Atomen und mindestens zwei C-C-Doppelbindungen, mittels Distickstoffmonoxid unter Erhalt einer Zusammensetzung (A1), mindestens enthaltend die mindestens eine cyclische Verbindung mit Z Cyclen und 7 bis 16 C-Atomen mit einer Ketogruppe, das mindestens eine cyclische Olefin mit Z Cyclen und 7 bis 16 C-Atomen mit mindestens zwei C-C-Doppelbindungen und mindestens eine Verbindung mit Z-1 Cyclen und 7 bis 16 C-Atomen mit mindestens einer Aldehydgruppe, (a2) Abtrennen des mindestens einen cyclischen Olefins mit Z Cyclen und 7 bis 16 C-Atomen mit mindestens zwei C-C-Doppelbindungen aus der Zusammensetzung (A1) aus Stufe (a1), um eine Zusammensetzung (A2) zu erhalten, mindestens enthaltend die mindestens eine cyclische Verbindung mit Z Cyclen und 7 bis 16 C-Atomen mit einer Ketogruppe und mindestens eine Verbindung mit Z-1 Cyclen und 7 bis 16 C-Atomen mit mindestens einer Aldehydgruppe, und (b) destillative Behandlung der Zusammensetzung (A2) aus Schritt (a2), um eine Zusammensetzung (B) zu erhalten, enthaltend die mindestens eine cyclische Verbindung mit Z Cyclen und 7 bis 16 C-Atomen mit einer Ketogruppe und weniger als 1,0 Gew.-% der mindestens einen Verbindung mit Z-1 Cyclen und 7 bis 16 C-Atomen mit mindestens einer Aldehydgruppe, wobei Z 1, 2, 3 oder 4 sein kann.

WO 2008/000757 A1 offenbart ein Verfahren zur Herstellung eines cyclischen Ketons mit 7 bis 16 C-Atomen, mindestens umfassend die Stufen (a) Oxidation einer Zusammensetzung (I), mindestens enthaltend ein cyclisches Olefin mit 7 bis 16 C-Atomen mit mindestens einer C-C-Doppelbindung, mittels Stickstoffmonoxid unter Erhalt einer Zusammensetzung (A), (b) Behandeln der Zusammensetzung (A) mit mindestens einer Base unter Erhalt einer Zusammensetzung (B). Dieses Dokument offenbart nicht, dass vor der Behandlung der Zusammensetzung (A) mit mindestens einer Base in Schritt (b) die aus der Oxidation erhaltene Zusammensetzung destillativ gereinigt werden soll.

WO 2008/000756 A1 offenbart ein Verfahren zur Herstellung eines cyclischen Ketons mit 7 bis 16 C-Atomen, mindestens umfassend die Stufen (a) Oxidation einer Zusammensetzung (I), mindestens enthaltend ein cyclisches Olefin mit 7 bis 16 C-Atomen mit mindestens einer C-C-Doppelbindung, mittels Distickstoffmonoxid unter Erhalt einer Zusammensetzung (A), (b) Behandeln der Zusammensetzung (A) mit mindestens einer Base unter Erhalt einer Zusammensetzung (B), (c) Hydrieren der Zusammensetzung (B) in Gegenwart mindestens eines Katalysators unter Erhalt einer Zusammensetzung (C), (d) Reinigen der Zusammensetzung (C), mindestens umfassend die Schritte (di) thermisches Behandeln der Zusammensetzung (C) mit mindestens einer Säure, oder mindestens einem Katalysator, der mindestens ein Übergangsmetall enthält, (dii) weiteres Aufreinigen durch ein Verfahren ausgewählt aus der Gruppe bestehend aus Destillation, Extraktion und Kristallisation. Auch dieses Dokument offenbart nicht, dass die aus der Oxidation erhaltene Reaktionsmischung destillativ aufgereinigt wird, um den Gehalt an spezifischen, während der Oxidation gebildeten, Nebenprodukten zu senken.

WO 2005/030690 A2 offenbart ein Verfahren zur Herstellung eines Ketons, insbesondere von Cyclodecanon, wobei Cyclododecatrien mit Distickstoffmonoxid unter Erhalt von Cyclododecadienon umgesetzt wird, und das erhaltene Cyclododecadienon insbesondere zu Cyclododecanon hydriert wird. Dieses Dokument offenbart kein Verfahren zur Herstellung von cyclischen Ketonen, bei dem die Reaktionsmischung, die aus der Oxidation mit Distickstoffmonoxid erhalten wird, destillativ aufgereinigt wird, um den Gehalt an spezifischen, während der Oxidation gebildeten Nebenprodukten zu senken.

WO 2008/000754 A1 offenbart ein Verfahren zur Reinigung einer Zusammensetzung (I), mindestens enthaltend ein cyclisches Keton mit 7 bis 16 C-Atomen, umfassend das thermische Behandeln der Zusammensetzung (I) mit einem Katalysator, der mindestens ein Übergangsmetall enthält, und weiteres Aufreinigen durch ein Verfahren ausgewählt aus Destillation, Extraktion und Kristallisation. Das genannte Dokument offenbart kein Verfahren zur Herstellung von cyclischen Ketonen mit 7 bis 16 C-Atomen, durch Oxidation eines entsprechenden Olefins mit Distickstoffmonoxid, bei dem das aus der Oxidation erhaltene Reaktionsgemisch destillativ aufgereinigt wird, um den Gehalt an spezifischen, während der Oxidation gebildeten Nebenprodukten zu senken.

Die aus dem Stand der Technik bekannten Verfahren zur Herstellung cyclischer Ketone sind bezüglich der Reinheit der hergestellten cyclischen Ketone noch verbesserungsbedürftig. Ein Nachteil der Verfahren des Standes der Technik ist beispielsweise, dass, falls die während der Oxidation gebildeten Nebenprodukte nicht vollständig beziehungsweise nahezu vollständig abgetrennt werden, diese in den nachfolgenden Schritten stören, beziehungsweise in Verbindungen überführt werden, die in einem abschließenden Reinigungsschritt, beispielsweise einer Destillation, nicht vom dem gewünschten Produkt abgetrennt werden können, da die in den cyclischen Ketonen enthaltenen Verunreinigungen, beispielsweise Aldehyde, durch herkömmliche Reinigungsverfahren wie Destillation, Extraktion oder Umkristallisation nur schwer entfernt werden, da die funktionellen Gruppen und die Anzahl der Kohlenstoffatome ähnlich sind. Dies ist insofern problematisch als cyclische Ketone für verschiedene Anwendungen in hoher Reinheit benötigt werden. So ist beispielsweise Cyclododecanon ein wichtiges Zwischenprodukt für die Herstellung von beispielsweise Lauryllactam, Dodecandicarbonsäure und daraus abgeleiteten Polyamide wie beispielsweise Nylon 12 oder Nylon 6.12. Deshalb ist in diesen Fällen eine sehr aufwendige Reinigung, beispielsweise durch vielstufige Destillation und/oder Kristallisation notwendig. Diese Reinigungsverfahren sind dadurch aufwendig und kostenintensiv.

Daher ist es eine Aufgabe der vorliegenden Erfindung, ein Verfahren bereitzustellen, mit dem cyclische Ketone mit 7 bis 16 Kohlenstoffatomen in einer besonders hohen Reinheit erhalten werden können. Eine weitere Aufgabe der vorliegenden Erfindung ist es, dass bereits bestehende Anlagen bzw. Apparaturen zur Herstellung von cyclischen Ketonen mit 7 bis 16 C-Atomen weiter genutzt werden können, um den zusätzlichen apparativen Aufbau möglichst gering zu halten.

Diese Aufgaben werden erfindungsgemäß gelöst durch ein
Verfahren zur Herstellung mindestens einer cyclischen Verbindung mit Z Cyclen und 7 bis 16 C-Atomen mit einer Ketogruppe, mindestens umfassend die Stufen:
(a1) Oxidation einer Zusammensetzung (A), mindestens enthaltend ein cyclisches Olefin mit Z Cyclen und 7 bis 16 C-Atomen und mindestens zwei C-C-Doppelbindungen, mittels Distickstoffmonoxid unter Erhalt einer Zusammensetzung (A1), mindestens enthaltend
   - die mindestens eine cyclische Verbindung mit Z Cyclen und 7 bis 16 C-Atomen mit einer Ketogruppe,
   - mindestens eine cyclische Verbindung mit Z Cyclen und 7 bis 16 C-Atomen mit mindestens zwei Ketogruppen,
   - das mindestens eine cyclische Olefin mit Z Cyclen und 7 bis 16 C-Atomen mit mindestens zwei C-C-Doppelbindungen und
   - mindestens eine Verbindung mit Z-1 Cyclen und 7 bis 16 C-Atomen mit mindestens einer Aldehydgruppe,
(a2) Abtrennen des mindestens einen cyclischen Olefins mit Z Cyclen und 7 bis 16 C-Atomen mit mindestens zwei C-C-Doppelbindungen aus der Zusammensetzung (A1) aus Stufe (a1), um eine Zusammensetzung (A2) zu erhalten, mindestens enthaltend
   - die mindestens eine cyclische Verbindung mit Z Cyclen und 7 bis 16 C-Atomen mit einer Ketogruppe,
   - mindestens eine cyclische Verbindung mit Z Cyclen und 7 bis 16 C-Atomen mit mindestens zwei Ketogruppen und
   - mindestens eine Verbindung mit Z-1 Cyclen und 7 bis 16 C-Atomen mit mindestens einer Aldehydgruppe, und
(b) destillative Behandlung der Zusammensetzung (A2) aus Schritt (a2), um eine Zusammensetzung (B) zu erhalten, enthaltend
   - die mindestens eine cyclische Verbindung mit Z Cyclen und 7 bis 16 C-Atomen mit einer Ketogruppe,
   - weniger als 0,5 Gew.-% der mindestens einen cyclischen Verbindung mit Z Cyclen und 7 bis 16 C-Atomen mit mindestens zwei Ketogruppen und
   - weniger als 1,0 Gew.-% der mindestens einen Verbindung mit Z-1 Cyclen und 7 bis 16 C-Atomen mit mindestens einer Aldehydgruppe, wobei sich an Stufe (b) mindestens die folgende Stufe (c) anschließt:
(c) Hydrierung der Zusammensetzung (B) in Gegenwart mindestens eines Katalysators, um eine Zusammensetzung (C) zu erhalten,
   oder sich an Stufe (b) mindestens die folgende Stufe (d) anschließt:
(d) Behandeln der Zusammensetzung (B) mit wenigstens einer Base, um eine Zusammensetzung (D) zu erhalten,
   und sich an Stufe (d) mindestens die folgende Stufe (c') anschließt:
(c') Hydrierung der Zusammensetzung (D) in Gegenwart mindestens eines Katalysators, um eine Zusammensetzung (C') zu erhalten, dadurch gekennzeichnet, dass das mindestens eine cyclische Olefin mit Z Cyclen und 7 bis 16 C-Atomen und mindestens zwei C-C-Doppelbindungen ausgewählt ist aus der Gruppe bestehend aus 1,5-Cyclooctadien, 1,5-Cyclododecadien, 1,9-Cyclohexadecadien, 1,8-Cyclotetradecadien, 1,6-Cyclododecadien, 1,6,11-Cyclopentadecatrien, 1,5,9-Cyclododecatrien, Vinylcyclohexen, Norbornadien, Ethylidennorbornen und Mischungen davon.

Es wurde überraschend gefunden, dass insbesondere Nebenprodukte wie cyclische und offenkettige Aldehyde, die einen Cyclus weniger (Z-1 Cyclen) aufweisen als die eingesetzten Ausgangsverbindungen, oder gegebenenfalls auch Nebenprodukte, die Ketogruppen enthalten, allgemein aus einem Gemisch mit cyclischen Ketonen mit derselben oder einer ähnlichen Anzahl an C-Atomen selektiv abgereichert werden können, indem die aus der Oxidation erhaltene Mischung nach Abtrennung von verbliebenem Edukt destillativ aufgereinigt wird, um den Gehalt dieser störenden Nebenprodukte zu senken. Durch die Entfernung der genannten Verbindungen direkt nach der Oxidation ist es möglich, dass gewünschte Endprodukt, die mindestens eine cyclische Verbindung mit Z Cyclen und 7 bis 16 C-Atomen mit einer Ketogruppe, in einer besonders hohen Reinheit zu erhalten, die ohne den erfindungsgemäßen Schritt (b) nicht zugänglich ist.

Des Weiteren kann durch das destillative Abtrennen der genannten Verbindungen in Schritt (b) des erfindungsgemäßen Verfahrens das Entstehen von störenden Verbindungen, die von dem gewünschten Produkt nicht vollständig abtrennbar sind, unterbunden werden.

Durch das erfindungsgemäße Verfahren können cyclische Verbindungen mit 7 bis 16 C-Atomen und einer Ketogruppe mit einer Reinheit von beispielsweise mindestens 95 %, bevorzugt mindestens 98 %, erhalten werden. Die Reinheit kann nach allen dem Fachmann bekannten Verfahren bestimmt werden, beispielsweise Gaschromatographie. Ein weiterer Vorteil des erfindungsgemäßen Verfahrens ist, dass es leicht mit bestehenden Anlagen kombiniert werden kann, so dass keine kostenintensiven Umbauten erforderlich sind.

### Stufe (a1)

Die Umsetzung gemäß Stufe (a1) kann generell gemäß sämtlichen Verfahrensführungen erfolgen, bei denen das Olefin und Distickstoffmonoxid miteinander reagieren.

In Stufe (a1) des erfindungsgemäßen Verfahrens wird das cyclische Olefin durch Umsetzung mit Distickstoffmonoxid oxidiert. Dabei kann für die Umsetzung des cyclischen Olefins mit Distickstoffmonoxid mindestens ein geeignetes Lösungsmittel oder Verdünnungsmittel eingesetzt werden. Als solche sind unter anderem cyclische Alkane, beispielsweise Cyclododecan oder Cyclododecanon oder gesättigte aliphatische oder aromatische, gegebenenfalls alkylsubstituierte Kohlenwasserstoffe zu nennen, wobei im Wesentlichen alle gängigen Lösungs- und/oder Verdünnungsmittel geeignet sind unter der Maßgabe, dass sie weder eine C-C-Doppelbindung, noch eine C-C-Dreifachbindung, noch eine Aldehydgruppe aufweisen.

Im Allgemeinen ist bei der Umsetzung des cyclischen Olefins mit Distickstoffmonoxid der Zusatz eines Lösungsmittels oder Verdünnungsmittels nicht notwendig.

Die Temperatur bei der Umsetzung des cyclischen Olefins mit Distickstoffmonoxid liegt bevorzugt bei 140 bis 350 °C, weiter bevorzugt bei 180 bis 320 °C und besonders bevorzugt bei 200 bis 300 °C.

Es ist möglich, die Umsetzung des cyclischen Olefins mit Distickstoffmonoxid bei zwei oder mehr Temperaturen beziehungsweise in zwei oder mehr Temperaturbereichen durchzuführen, die jeweils in den oben angegebenen Grenzen liegen. Temperaturänderungen im Laufe der Umsetzung können kontinuierlich oder auch diskontinuierlich vollzogen werden.

Der Druck bei der Umsetzung des cyclischen Olefins mit Distickstoffmonoxid liegt bevorzugt höher als der Eigendruck des Edukt- bzw. Produktgemisches bei der gewählten Reaktionstemperatur oder den gewählten Reaktionstemperaturen. Der Druck liegt bevorzugt bei 1 bis 1000 bar, weiter bevorzugt bei 40 bis 325 bar und besonders bevorzugt bei 50 bis 200 bar.

Es ist möglich, die Umsetzung des cyclischen Olefins mit Distickstoffmonoxid bei zwei oder mehr Drücken beziehungsweise in zwei oder mehr Druckbereichen durchzuführen, die jeweils in den oben angegebenen Grenzen liegen. Druckänderungen im Laufe der Umsetzung können kontinuierlich oder auch diskontinuierlich vollzogen werden.

Hinsichtlich der für die Umsetzung des cyclischen Olefins mit Distickstoffmonoxid einsetzbaren Reaktoren existieren keine besonderen Beschränkungen. Insbesondere kann die Umsetzung in Batch-Fahrweise oder in kontinuierlicher Fahrweise erfolgen. Demgemäß können beispielsweise als Reaktoren mindestens ein CSTR (Continuous Stirred Tank Reactor) mit mindestens einem internen und/oder mindestens einem externen Wärmetauscher, mindestens ein Rohrreaktor, mindestens ein Rohrbündelreaktor oder mindestens ein Schlaufenreaktor eingesetzt werden. Ebenso ist es möglich, mindestens einen dieser Reaktoren so auszugestalten, dass er mindestens zwei unterschiedliche Zonen aufweist. Solche Zonen können sich beispielsweise in Reaktionsbedingungen wie beispielsweise der Temperatur oder dem Druck und/oder in der Geometrie der Zone wie beispielsweise dem Volumen oder dem Querschnitt unterscheiden. Wird die Umsetzung in zwei oder mehr Reaktoren durchgeführt, können zwei oder mehr gleiche Reaktortypen oder mindestens zwei verschiedene Reaktortypen eingesetzt werden.

Bevorzugt wird die Umsetzung des cyclischen Olefins mit Distickstoffmonoxid in einem einzigen Reaktor durchgeführt. Beispielsweise bevorzugt ist die Umsetzung in kontinuierlicher Fahrweise. Ein geeigneter Reaktor ist beispielsweise in der noch nicht veröffentlichten Patentanmeldung EP 09151002.4 beschrieben.

Die Verweilzeit des Reaktionsgutes in dem mindestens einen Reaktor bei der Umsetzung des cyclischen Olefins mit Distickstoffmonoxid liegt im allgemeinen im Bereich von bis zu 20 Stunden, bevorzugt im Bereich von 0,1 bis 20 Stunden, weiter bevorzugt im Bereich von 0,2 bis 15 Stunden und besonders bevorzugt im Bereich von 0,25 bis 10 Stunden.

Im Feed, der der Umsetzung von Distickstoffmonoxid mit dem cyclischen Olefin zugeführt wird, liegt das Molverhältnis von Distickstoffmonoxid und dem cyclischen Olefin im Allgemeinen im Bereich von 0,05 bis 4, bevorzugt im Bereich von 0,06 bis 1, weiter bevorzugt im Bereich von 0,07 bis 0,5 und besonders bevorzugt im Bereich von 0,1 bis 0,4.

Die Umsetzung des cyclischen Olefins mit Z Cyclen und 7 bis 16 C-Atomen mit mindestens zwei C-C-Doppelbindungen mit Distickstoffmonoxid kann so durchgeführt werden, dass bei einer sehr hohen Selektivität bezüglich der wenigstens einen cyclischen Verbindung mit Z Cyclen und 7 bis 16 C-Atomen mit einer Ketogruppe ein Umsatz des cyclischen Olefins im Bereich von bis zu 50%, bevorzugt im Bereich von 5 bis 30% und insbesondere bevorzugt im Bereich von 10 bis 20% erreicht wird. Dabei liegt die Selektivität, bezogen auf die mindestens eine cyclische Verbindung mit Z Cyclen 7 bis 16 C-Atomen und einer Ketogruppe, im Allgemeinen bei mindestens 90%, bevorzugt bei mindestens 92,5% und besonders bevorzugt bei mindestens 93%.

Die vorliegende Erfindung betrifft das erfindungsgemäße Verfahren wobei das mindestens eine cyclische Olefin mit zwei oder mehreren C-C-Doppelbindungen ausgewählt ist aus der Gruppe bestehend aus 1,5-Cyclooctadien, 1,5-Cyclododecadien, 1,9-Cyclohexadecadien, 1,8-Cyclotetradecadien, 1,6-Cyclodecadien, 1,6,11-Cyclopentadecatrien, 1,5,9-Cyclododecatrien, Vinylcyclohexen und Mischungen davon. Diese genannten erfindungsgemäß einsetzbaren Olefine weisen einen Cyclus auf, somit ist in diesen Fällen Z gleich eins. Weitere bevorzugte Verbindungen sind Norbornadien und Ethylidennorbornen, in denen Z gleich zwei ist.

1,5-Cyclooctadien (I), 1,5-Cyclododecadien (II), 1,9-Cyclohexadecadien (III), 1,8-Cyclotetradecadien (IV), 1,6-Cyclododecadien (V), 1,6,11-Cyclopentadecatrien (VI), 1,5,9-Cyclododecatrien (VII), 1,5,9,13-Cyclohexadecatetraen (VII), Norbornadien (IX), Ethylidennorbornen (X), Vinylcyclohexen (XI), wobei immer nur eines der möglichen Isomere dargestellt ist, sind im Folgenden abgebildet:

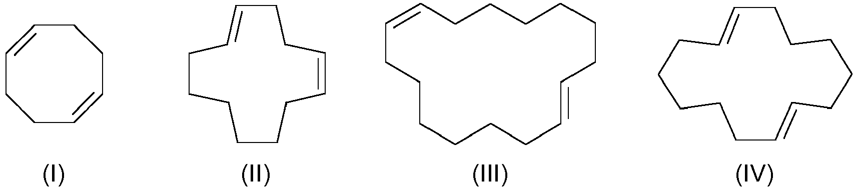

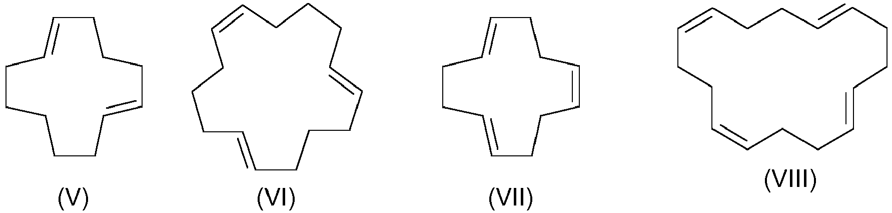

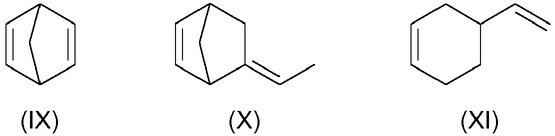

Besonders bevorzugt wird als cyclisches Olefin 1,5,9-Cyclododecatrien (VII) eingesetzt. 1,5,9-Cyclododecatrien kann im Allgemeinen in jedem möglichen Isomer eingesetzt werden, beispielsweise cis,trans,trans-1,5,9-Cyclododecatrien, cis,cis,trans-1,5,9-Cyclododecatrien, all-trans-1,5,9-Cyclododecatrien oder all-cis-1,5,9-Cyclododecatrien, ganz besonders bevorzugt cis,trans,trans-1,5,9-Cyclododecatrien. In dem erfindungsgemäßen Verfahren kann auch eine Mischung der genannten Isomere umgesetzt werden und insbesondere ein Isomerengemisch, das überwiegend cis,trans,trans-1,5,9-Cyclododecatrien enthält.

Daher betrifft die vorliegende Erfindung in einer bevorzugten Ausführungsform ein Verfahren zur Herstellung eines Ketons wie oben beschrieben, wobei das cyclische Olefin mit Z Cyclen und 7 bis 16 C-Atomen mit mindestens zwei Doppelbindungen Cyclododecatrien, bevorzugt 1,5,9-Cyclododecatrien, besonders bevorzugt cis,trans,trans-1,5,9-Cyclododecatrien, ist.

Das in dem erfindungsgemäßen Verfahren vorzugsweise eingesetzte Cyclododecatrien kann im Allgemeinen durch alle dem Fachmann bekannte Verfahren erhalten werden, In einer bevorzugten Ausführungsform wird das Cyclododecatrien durch Trimerisierung von Butadien erhalten.

Daher betrifft die vorliegende Erfindung gemäß einer weiteren Ausführungsform auch ein wie zuvor beschriebenes Verfahren zur Herstellung eines cyclischen Ketons mit Z Cyclen und 7 bis 16 C-Atomen mit einer Ketogruppe, wobei das cyclische Olefin mit Z Cyclen und 7 bis 16 C-Atomen mit mindestens zwei Doppelbindungen Cyclododecatrien ist, das mittels Trimerisierung aus Butadien hergestellt wurde.

1,5,9-Cyclododecatrien kann beispielsweise durch Trimerisierung von reinem 1,3-Butadien hergestellt werden, wie dies beispielsweise in T. Schiffer, G. Oenbrink, "Cyclododecatriene, Cyclooctadiene, and 4-Vinylcyclohexene", in Ullmann's Encyclopedia of Industrial Chemistry, 6th Edition (2000), Electronic Release, Wiley VCH, Seiten 1 bis 4, beschrieben ist. Im Rahmen dieses Verfahrens entstehen beispielsweise bei der Trimerisierung in Anwesenheit von Ziegler-Katalysatoren cis,trans,trans-1,5,9-Cyclododecatrien, cis,cis,trans-1,5,9-Cyclododecatrien und all-trans-1,5,9-Cyclododecatrien, wie dies beispielsweise in H. Weber et al. "Zur Bildungsweise von cis,trans,trans-Cyclododecatrien-(1.5.9) mittels titanhaltiger Katalysatoren" in Liebigs Ann. Chem. 681 (1965) Seiten 10 bis 20 beschrieben ist. Cyclododecatrien kann beispielsweise durch Trimerisierung von 1,3-Butadien unter Verwendung eines Titan- oder NickelKatalysators, beispielsweise gemäß DE 1283836, hergestellt werden.

Während grundsätzlich sämtliche geeigneten Titan-Katalysatoren zur Trimerisierung eingesetzt werden können, ist der im Artikel von Weber et al. beschriebene Titantetrachlorid/Ethylaluminiumsesquichlorid-Katalysator besonders geeignet.

Während grundsätzlich sämtliche geeigneten Nickel-Katalysatoren zur Trimerisierung eingesetzt werden können, ist der in der DE 1283836 beschriebene bis-Cyclooctadienylnickel/Ethoxydiethylaluminium-Katalysator besonders geeignet.

Das für die Trimerisierung eingesetzte Butadien weist insbesondere bevorzugt einen über Gaschromatographie bestimmten Reinheitsgrad von mindestens 99,6% und weiter bevorzugt von mindestens 99,65% auf. Insbesondere bevorzugt enthält das eingesetzte 1,3-Butadien im Rahmen der Nachweisgenauigkeit kein 1,2-Butadien und kein 2-Butin.

Aus dieser Trimerisierung werden im Allgemeinen Gemische erhalten, die mindestens 95 Gew.-%, bevorzugt mindestens 96 Gew.-% und weiter bevorzugt mindestens 97 Gew.-% cis,trans,trans-1,5,9-Cyclododecatrien enthalten. Insbesondere bevorzugt enthalten die Gemische in etwa 98 Gew.-% cis,trans,trans-1,5,9-Cyclododecatrien.
Dieses cis,trans,trans-1,5,9-Cyclododecatrien enthaltende Gemisch kann als solches für die erfindungsgemäße Umsetzung gemäß Stufe (a) verwendet werden. Ebenso ist es möglich, über mindestens eine geeignete Methode, beispielsweise bevorzugt über mindestens eine Destillation, das cis,trans,trans-1,5,9-Cyclododecatrien aus dem Gemisch abzutrennen und in der Umsetzung gemäß Stufe (a) einzusetzen.

Gemäß einer ganz besonders bevorzugten Ausführungsform des erfindungsgemäßen Verfahrens wird als Cyclododecatrien ein Isomerengemisch eingesetzt, das überwiegend cis,trans,trans-1,5,9-Cyclododecatrien, trans,trans,trans-1,5,9-Cyclododecatrien oder cis,cis,trans-1,5,9-Cyclododecatrien enthält. Vorzugsweise wird ein Isomerengemisch eingesetzt, das mehr als 60 Gew.-%, bezogen auf das Isomerengemisch, cis,trans,trans-1,5,9-Cyclododecatrien enthält, weiter bevorzugt mehr als 70 Gew.-%, insbesondere mehr als 80 Gew.-%, besonders bevorzugt mehr als 90 Gew.-%, beispielsweise mehr als 91 Gew.-%, mehr als 92 Gew.-%, mehr als 93 Gew.-%, mehr als 94 Gew.-%, mehr als 95 Gew.-%, mehr als 96 Gew.-%, mehr als 97 Gew.-% oder mehr als 98 Gew.-%.

Die erfindungsgemäß einsetzbaren Olefine können beispielsweise durch die in den folgenden Literaturstellen genannten Verfahren hergestellt werden:
(I) Cycloocta-1,5-dien fällt als Nebenprodukt bei der Synthese von Verbindung (VII) an, wie dies beispielsweise in T. Schiffer, G. Oenbrink, "Cyclododecatriene, Cyclooctadiene, and 4-Vinylcyclohexene", in Ullmann's Encyclopedia of Industrial Chemistry, 6th Edition (2000), Electronic Release, Wiley VCH beschrieben ist.
(II) Cyclododeca-1,5-dien kann zum Beispiel durch katalytische Reduktion von Verbindung (VII) gewonnen werden, wie zum Beispiel in der US 3,182,093 beschrieben wird.
(III) Cyclohexadeca-1,9-dien kann durch Metathese von Cycloocten gewonnen werden, wie zum Beispiel in EP 1288181 beschrieben wird.
(IV) Cyclotetradeca-1,8-dien kann durch Metathese von Cyclohepten gewonnen werden, wie zum Beispiel in S. Warwel, H. Kaetker, Synthesis (1987) (10), 935-7 beschrieben wird.
(V) Cyclodeca-1,6-dien, bevorzugt das cis,cis-Isomer, kann durch Isomerisierung von cis,trans-Cyclodeca-1,5-dien gewonnen werden, wie zum Beispiel in der DE 1 230 023 beschrieben wird.
(VI) Cyclopentadecadeca-1,6,11-trien kann durch Cyclooligomerisierung von Cyclopenten gewonnen werden, wie zum Beispiel in der DD 115480 beschrieben wird.
(VII) Siehe (I)
(VIII) Cyclohexadeca-1,5,9,13-tetraen kann durch Tetramerisierung von Butadien gewonnen werden, wie zum Beispiel in U. M. Dzhemilev, L. Yu. Gubaidullin, G. A. Tolstikov, Zhurnal Organicheskoi Khimii (1976), 12(1), 44-6 beschrieben wird.
(IX) Norbornadien kann durch Umsetzung von Cyclopentadien mit Acetylen gewonnen werden, wie zum Beispiel in der US 2875256 beschrieben wird.
(X) Ethylidennorbornen kann durch basenkatalysierte Umlagerung von 5-Vinyl-2-norbornen gewonnen werden, wie zum Beispiel in der EP 0 279 397 beschrieben wird.
(XI) 4-Vinylcyclohexen kann durch Diels-Alder-Reaktion von Butadien mit sich selbst hergestellt werden, fällt aber auch als Nebenprodukt bei der Herstellung von Verbindung (VII) an, wie zum Beispiel in T. Schiffer, G. Oenbrink, "Cyclododecatriene, Cyclooctadiene, and 4-Vinylcyclohexene", in Ullmann's Encyclopedia of Industrial Chemistry, 6th Edition (2000), Electronic Release, Wiley VCH, Seiten 1-4, beschrieben ist.

Aus der erfindungsgemäßen Umsetzung von Zusammensetzung (A), mindestens enthaltend ein cyclisches Olefin mit Z Cyclen und 7 bis 16 C-Atomen und mindestens zwei C-C-Doppelbindungen, mittels Distickstoffmonoxid wird eine Zusammensetzung (A1) erhalten, mindestens enthaltend
- die mindestens eine cyclische Verbindung mit Z Cyclen und 7 bis 16 C-Atomen mit einer Ketogruppe,
- das mindestens eine cyclische Olefin mit Z Cyclen und 7 bis 16 C-Atomen mit mindestens zwei C-C-Doppelbindungen und
- mindestens eine Verbindung mit Z-1 Cyclen und 7 bis 16 C-Atomen mit mindestens einer Aldehydgruppe,

Im Allgemeinen resultiert aus der bevorzugten erfindungsgemäßen Umsetzung von cis,trans,trans-1,5,9-Cyclododecatrien mit Distickstoffmonoxid gemäß Stufe (a1) ein Cyclododeca-4,8-dienon-lsomerengemisch, das mindestens zwei der Isomere cis,trans-Cyclododeca-4,8-dienon, trans,cis-Cyclododeca-4,8-dienon und trans,trans-Cyclododeca-4,8-dienon als cyclische Verbindungen mit Z Cyclen und 7 bis 16 C-Atomen mit einer Ketogruppe enthält.

Bevorzugt wird erfindungsgemäß ein Isomerengemisch erhalten, bei dem trans,cis- und cis,trans-Isomere in etwa gleichen Mengen gebildet werden, und das trans,trans-Isomer im Vergleich zu den beiden anderen Isomeren in nur geringen Mengen gebildet wird. Ein beispielsweise typisches Isomerengemisch weist demgemäß die genannten Isomeren in molaren Verhältnissen von in etwa 1 : 1 : 0,08 auf.

Die in Zusammensetzung (A1) enthaltene mindestens eine cyclische Verbindung mit Z Cyclen und 7 bis 16 C-Atomen mit einer Ketogruppe ist das gewünschte Produkt von Stufe (a) des erfindungsgemäßen Verfahrens. Da bevorzugt als Substrat mindestens eine cyclische Verbindung mit Z Cyclen und 7 bis 16 C-Atomen und drei Doppelbindungen eingesetzt wird, wird als besonders bevorzugtes Produkt in Stufe (a) des erfindungsgemäßen Verfahrens durch Oxidation einer dieser Doppelbindungen mindestens eine cyclische Verbindung mit Z Cyclen und 7 bis 16 C-Atomen mit zwei Doppelbindungen und einer Ketogruppe gebildet. In einer weiteren bevorzugten Ausführungsform wird dieses Produkt aus Stufe (a) durch eine Hydrierung in einer noch folgenden Stufe in mindestens eine gesättigte cyclische Verbindung mit Z Cyclen und 7 bis 16 C-Atomen und einer Ketogruppe, insbesondere Cyclododecanon, überführt.

Die Zusammensetzung (A1) enthält im Rahmen der vorliegenden Erfindung mindestens eine cyclische Verbindung mit Z Cyclen und 7 bis 16 C-Atomen mit einer Ketogruppe in einer Menge von im Allgemeinen mehr als 5 Gew.-%, bevorzugt mehr als 10 Gew.-%, vorzugsweise 10 bis 90 Gew.-%, insbesondere 11 bis 50 Gew.-%, besonders bevorzugt 12 bis 40 Gew.-%, insbesondere bevorzugt 13 bis 30 Gew.-%, beispielsweise 14 bis 20 Gew.-% oder 15 bis 18 Gew.-%.

Erfindungsgemäß liegen in Zusammensetzung (A1) auch mindestens
- das mindestens eine cyclische Olefin mit Z Cyclen und 7 bis 16 C-Atomen mit mindestens zwei C-C-Doppelbindungen und
- mindestens eine Verbindung mit Z-1 Cyclen und 7 bis 16 C-Atomen mit mindestens einer Aldehydgruppe,
vor.

Das mindestens eine cyclische Olefin mit Z Cyclen und 7 bis 16 C-Atomen mit mindestens zwei C-C-Doppelbindungen, die in Zusammensetzung (A1) vorliegt, ist die gleiche Verbindung, die in Zusammensetzung (A) vor der Oxidation als Ausgangsverbindung eingesetzt worden ist. Das mindestens eine cyclische Olefin mit Z Cyclen und 7 bis 16 C-Atomen mit mindestens zwei C-C-Doppelbindungen in Zusammensetzung (A1) ist somit verbliebenes Edukt, welches in Stufe (a1) des erfindungsgemäßen Verfahrens nicht oxidiert worden ist. Erfindungsgemäß kann das mindestens eine cyclische Olefin mit Z Cyclen und 7 bis 16 C-Atomen mit mindestens zwei Doppelbindungen in Zusammensetzung (A1) in den gleichen isomeren Strukturen vorliegen, in denen es als Edukt eingesetzt worden ist. In einer bevorzugten Ausführungsform liegt in Zusammensetzung (A1) bezüglich des mindestens einen Olefins mit Z Cyclen und 7 bis 16 C-Atomen mit mindestens zwei C-C-Doppelbindungen ein etwas anderes Isomerenverhältnis vor, als in dem in Schritt (a1) eingesetzten Edukt. Dies ist beispielsweise in den verschiedenen Reaktivitäten der einzelnen Isomeren begründet, beispielsweise reagiert das all-trans-Isomer schneller als das cis,trans,trans-Isomer, und dieses wiederum etwas schneller als das cis,cis,trans-Isomer.

Die in der Zusammensetzung (A1) vorliegende mindestens eine Verbindung mit Z-1 Cyclen und 7 bis 16 C-Atomen mit mindestens einer Aldehydgruppe entsteht aus dem als Edukt eingesetzten Olefin mit mindestens zwei Doppelbindungen durch oxidative Spaltung einer Doppelbindung.

Da, wie oben beschrieben, Z die Anzahl der in den genannten Verbindungen vorliegenden Cyclen beschreibt, bedeutet die Formulierung "Z-1 Cyclen", dass in den so beschriebenen Verbindungen ein Cyclus weniger vorliegt, als in den Verbindungen, die Z Cyclen enthalten. Eine Verbindung kann erfindungsgemäß beispielsweise durch eine Ringöffnungsreaktion einen Cyclus weniger als das Edukt enthalten.

In Stufe (a1) des erfindungsgemäßen Verfahrens wird auch mindestens eine Verbindung mit Z Cyclen und 7 bis 16 C-Atomen mit mindestens einer Aldehydgruppe als Nebenprodukt gebildet. Dieses mindestens eine Nebenprodukt weist die gleiche Anzahl an Cyclen wie das in Stufe (a1) eingesetzte Edukt auf. Bei dieser mindestens eine Verbindung handelt es sich bevorzugt um eine cyclische Verbindung mit mindestens einer Aldehydgruppe die durch Ringverengung entsteht.

Die mindestens eine Verbindung mit Z-1 Cyclen und 7 bis 16 C-Atomen mit mindestens einer Aldehydgruppe liegt in der Zusammensetzung (A) im Allgemeinen in einer Menge von 0,1 bis 50,0 Gew.-%, bevorzugt 0,5 bis 10,0 Gew.-%, besonders bevorzugt 1,0 bis 5,0 Gew.-%, vor.

Für den bevorzugten Fall, dass in dem erfindungsgemäßen Verfahren als Edukt 1,5,9-Cyclododecatrien, insbesondere cis,trans,trans-1,5,9-Cyclododecatrien (Z=1), eingesetzt wird, wird als mindestens eine Verbindung mit Z-1 Cyclen und 7 bis 16 C-Atomen mit mindestens einer Aldehydgruppe besonders bevorzugt ein Isomerengemisch von acyclischen Verbindungen mit einer Aldehydgruppe und drei Doppelbindungen erhalten, beispielsweise eine Mischung von 4,8,11-Dodecatrienalen, wie eine Mischung von cis,trans-, trans,cis-, trans,trans-Isomeren, beispielsweise im ungefähren Verhältnis von 50 : 45 : 5. Das cis,trans-Isomer ist als Verbindung (XIII) abgebildet

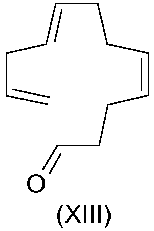

Für diesen bevorzugten Fall mit Z gleich eins für das Edukt weist die mindestens eine Verbindung mit Z-1 Cyclen und 7 bis 16 C-Atomen und mindestens einer Aldehydfunktion keinen Cyclus auf, da für Z-1 gilt: 1 -1 = 0.

Die in Stufe (a1) erhaltene Zusammensetzung (A1) enthält zusätzlich mindestens eine cyclische Verbindung mit Z Cyclen und 7 bis 16 C-Atomen mit mindestens zwei Ketogruppen. Diese Verbindung entsteht aus dem als Edukt eingesetzten Olefin mit mindestens zwei Doppelbindungen durch Oxidation von zwei der vorliegenden Doppelbindungen mit Distickstoffmonoxid. Diese mindestens eine cyclische Verbindung mit Z Cyclen und 7 bis 16 C-Atomen mit mindestens zwei Ketogruppen liegt in einer bevorzugten Ausführungsform in der Zusammensetzung (A1) im Allgemeinen in einer Menge von 0,1 bis 20,0 Gew.-%, bevorzugt 0,5 bis 10,0 Gew.-%, besonders bevorzugt 1,0 bis 5,0 Gew.-%, vor.

Für den besonders bevorzugten Fall, dass in das erfindungsgemäße Verfahren als Edukt 1,5,9-Cyclododecatrien, insbesondere cis,trans,trans-1,5,9-Cyclododecatrien, eingesetzt wird, wird als mindestens eine cyclische Verbindung mit Z Cyclen und 7 bis 16 C-Atomen mit mindestens zwei Ketogruppen besonders bevorzugt ein Isomerengemisch von cyclischen Verbindungen mit zwei Ketofunktionen und einer Doppelbindung erhalten, insbesondere Cyclododecendione, beispielsweise eine Mischung der 8-cis-Cyclododecen-1,5-dion-, 9-cis-Cyclododecen-1,6-dion-, 8-cis-Cyclododecen-1,4-dion-, 8-trans-Cyclododecen-1,5-dion-, 8-trans-Cyclododecen-1,4-dion- und 9-trans-Cyclododecen-1,6-dion-Isomere, beispielsweise im ungefähren Verhältnis von 38 : 19 : 19 : 12 : 6 : 6. 8-cis-Cyclododecen-1,5-dion-Isomer, das als Hauptisomer gebildet wird, ist als Verbindung (XII) abgebildet

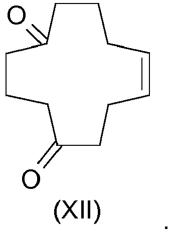

Die vorliegende Erfindung betrifft somit in einer bevorzugten Ausführungsform das erfindungsgemäße Verfahren, wobei es mindestens folgende Stufen umfasst:
(a1) Oxidation einer Zusammensetzung (A), mindestens enthaltend ein cyclisches Olefin mit Z Cyclen und 7 bis 16 C-Atomen und mindestens zwei C-C-Doppelbindungen, mittels Distickstoffmonoxid unter Erhalt einer Zusammensetzung (A1), mindestens enthaltend
   - die mindestens eine cyclische Verbindung mit Z Cyclen und 7 bis 16 C-Atomen mit einer Ketogruppe,
   - mindestens eine cyclische Verbindung mit Z Cyclen und 7 bis 16 C-Atomen mit mindestens zwei Ketogruppen,
   - das mindestens eine cyclische Olefin mit Z Cyclen und 7 bis 16 C-Atomen mit mindestens zwei C-C-Doppelbindungen und
   - mindestens eine Verbindung mit Z-1 Cyclen und 7 bis 16 C-Atomen mit mindestens einer Aldehydgruppe,
(a2) Abtrennen des mindestens einen cyclischen Olefins mit Z Cyclen und 7 bis 16 C-Atomen mit mindestens zwei C-C-Doppelbindungen aus der Zusammensetzung (A1) aus Stufe (a1), um eine Zusammensetzung (A2) zu erhalten, mindestens enthaltend
   - die mindestens eine cyclische Verbindung mit Z Cyclen und 7 bis 16 C-Atomen mit einer Ketogruppe,
   - mindestens eine cyclische Verbindung mit Z Cyclen und 7 bis 16 C-Atomen mit mindestens zwei Ketogruppen und
   - mindestens eine Verbindung mit Z-1 Cyclen und 7 bis 16 C-Atomen mit mindestens einer Aldehydgruppe, und
(b) destillative Behandlung der Zusammensetzung (A2) aus Schritt (a2), um eine Zusammensetzung (B) zu erhalten, enthaltend
   - die mindestens eine cyclische Verbindung mit Z Cyclen und 7 bis 16 C-Atomen mit einer Ketogruppe,
   - weniger als 0,3 Gew.-% der mindestens einen cyclischen Verbindung mit Z Cyclen und 7 bis 16 C-Atomen mit mindestens zwei Ketogruppen und
   - weniger als 1,0 Gew.-% der mindestens einen Verbindung mit Z-1 Cyclen und 7 bis 16 C-Atomen mit mindestens einer Aldehydgruppe,
   wobei sich an Stufe (b) mindestens die Stufe (c),
   oder sich an Stufe (b) mindestens die Stufe (d) anschließt, wobei sich an Stufe (d) mindestens die Stufe (c') anschließt,
   und wobei diese Stufen, sowie das mindestens eine Cycloolefin, wie oben definiert sind.

Neben dem genannten gewünschten Produkten, genannten Nebenprodukten und nicht umgesetztem Edukt enthält die Zusammensetzung (A1) üblicherweise weitere Verbindungen, insbesondere organische Verbindungen, beispielsweise organische Verbindungen mit sauerstoffhaltigen Gruppen, beispielsweise Alkohole, Aldehyde oder Epoxide. Dabei können die organischen Verbindungen insbesondere dieselbe Anzahl oder eine abweichende Anzahl an C-Atomen aufweisen, wie das in der Zusammensetzung (A1) enthaltene cyclische Keton. In der Zusammensetzung (A1) können zusätzlich zu den genannten Komponenten nicht umgesetztes Distickstoffmonoxid und gebildeter Stickstoff vorliegen.

Im Rahmen der vorliegenden Erfindung kann in Stufe (a1) Distickstoffmonoxid in reiner Form oder in Form eines Gasgemisches enthaltend Distickstoffmonoxid eingesetzt werden.

Grundsätzlich kann in Stufe (a1) des erfindungsgemäßen Verfahrens jedes Gasgemisch enthaltend Distickstoffmonoxid eingesetzt werden. Erfindungsgemäß ist es auch möglich, das Gasgemisch enthaltend Distickstoffmonoxid vor Einsatz in Stufe (a) zu reinigen oder aufzukonzentrieren. Ein geeignetes Reinigungsverfahren umfasst beispielsweise die Absorption des Gasgemisches in einem organischen Lösungsmittel oder Wasser, die Desorption des Gasgemisches aus dem beladenen organischen Lösungsmittel oder dem beladenen Wasser und das Einstellen des Gehalts an Stickoxiden NOₓ in dem Gasgemisch auf höchstens 0,01 bis 0,001 Vol.-%, bezogen auf das Gesamtvolumen des Gasgemisches. Ein derartiges Verfahren ist beispielsweise in DE 10 2004 046 167.8 beschrieben, deren diesbezüglicher Inhalt vollumfänglich in den Kontext der vorliegenden Anmeldung aufgenommen wird.

Dabei kann das eingesetzte Gasgemisch enthaltend Distickstoffmonoxid grundsätzlich aus jeder beliebigen Quelle stammen. Insbesondere ist es möglich, dass als Distickstoffmonoxidquelle das Abgas eines Verfahrens eingesetzt wird wie in WO 2006/032502, WO 2007/060160 und WO 2008/071632, und in den noch nicht veröffentlichten Anmeldungen EP 08153953.8 und EP 08153952.0 beschrieben.

Der Begriff "Gasgemisch", wie er im Rahmen der vorliegenden Erfindung verwendet wird, bezeichnet ein Gemisch aus zwei oder mehr Verbindungen, die sich bei Umgebungsdruck und Umgebungstemperatur im gasförmigen Zustand befinden. Bei veränderter Temperatur oder verändertem Druck kann das Gasgemisch auch in einem anderen Aggregatzustand vorliegen, beispielsweise flüssig, und wird im Rahmen der vorliegenden Erfindung weiter als Gasgemisch bezeichnet.

Erfindungsgemäß kann auch ein Gemisch verschiedener Abgase eingesetzt werden.

Gemäß einer weiter bevorzugten Ausführungsform der vorliegenden Erfindung stammt das mindestens eine Distickstoffmonoxid enthaltende Abgas aus einer Adipinsäureanlage, einer Dodecandisäureanlage, einer Hydroxylaminanlage und/oder einer Salpetersäureanlage, wobei letztere wiederum bevorzugt mit mindestens einem Abgas einer Adipinsäureanlage, einer Dodecandisäureanlage, einer Glyoxalanlage oder einer Hydroxylaminanlage betrieben wird.

Erfindungsgemäß kann das Gasgemisch gasförmig eingesetzt werden. Es ist jedoch auch möglich, das Gasgemisch enthaltend Distickstoffmonoxid zunächst derart zu behandeln, dass das Gasgemisch bzw. Distickstoffmonoxid in flüssiger oder überkritischer Form vorliegt und dann eingesetzt wird. Das Gasgemisch bzw. Distickstoffmonoxid kann durch geeignete Wahl des Drucks oder der Temperatur verflüssigt werden. Ebenso ist es im Rahmen der vorliegenden Erfindung möglich, das Gasgemisch in ein Lösungsmittel einzulösen.

Die Umsetzung des mindestens einen cyclischen Olefins mit Z Cyclen und 7 bis 16 C-Atomen und mindestens zwei C-C-Doppelbindungen mit Distickstoffmonoxid gemäß Schritt (a1) kann grundsätzlich in Anwesenheit eines Katalysators, jedoch auch ohne Zusatz eines Katalysators, erfolgen.

Die in Stufe (a1) erhaltene Zusammensetzung (A1) wird im Anschluss an Stufe (a1) in der erfindungsgemäßen Stufe (a2) behandelt.

In einer bevorzugten Ausführungsform wird die aus Stufe (a1) erhaltene Zusammensetzung (A1) in einer Stufe (a1b) entspannt, um noch vorhandene gasförmige Edukte oder Produkte, beispielsweise nicht umgesetztes N₂O oder gebildetes N₂, zu entfernen, bevor Zusammensetzung (A1) in Stufe (a2) eingesetzt wird. Das Entspannen kann nach dem Fachmann bekannten Verfahren erfolgen, beispielsweise durch Überführen der Zusammensetzung (A1) in einen Raum, in dem ein niedrigerer Druck herrscht.

Somit umfasst das erfindungsgemäße Verfahren bevorzugt einen Schritt (a1b)
(a1b) Entspannen der Zusammensetzung (A1), um Distickstoffmonoxid und Stickstoff zu entfernen, um eine Zusammensetzung (A1) zu erhalten, die im Wesentlichen frei von Distickstoffmonoxid und Stickstoff ist.

### Stufe (a2):

(a2) Abtrennen des mindestens einen cyclischen Olefins mit Z Cyclen und 7 bis 16 C-Atomen mit mindestens zwei C-C-Doppelbindungen aus der Zusammensetzung (A1) aus Stufe (a1), um eine Zusammensetzung (A2) zu erhalten, mindestens enthaltend
   - die mindestens eine cyclische Verbindung mit Z Cyclen und 7 bis 16 C-Atomen mit einer Ketogruppe und
   - mindestens eine Verbindung mit Z-1 Cyclen und 7 bis 16 C-Atomen mit mindestens einer Aldehydgruppe.

In Stufe (a2) des erfindungsgemäßen Verfahrens wird Edukt des erfindungsgemäßen Verfahrens, welches in der Oxidationsreaktion in Stufe (a1) nicht umgesetzt worden ist, von der Zusammensetzung (A1) abgetrennt, um die Zusammensetzung (A2) zu erhalten.

Stufe (a2) kann nach allen dem Fachmann bekannten Methoden erfolgen. In einer bevorzugten Ausführungsform wird in Stufe (a2) des erfindungsgemäßen Verfahrens eine Destillation durchgeführt, um beispielsweise nicht umgesetztes Edukt, d. h. wenigstens ein cyclisches Olefin mit Z Cyclen und 7 bis 16 C-Atomen mit wenigstens zwei C-C-Doppelbindungen, aus dem Produktstrom abzutrennen, um dieses bevorzugt in Stufe (a1) des erfindungsgemäßen Verfahrens rückzuführen.

In einer bevorzugten Ausführungsform wird für die Destillation in Stufe (a2) eine einfache Destillationskolonne mit dem Fachmann bekannten Packungen verwendet. Die Destillation in Stufe (a2) des erfindungsgemäßen Verfahrens wird bevorzugt im Vakuum, beispielsweise bei einem Druck ≤ 1000 mbar, bevorzugt ≤ 500 mbar, besonders bevorzugt ≤ 300 mbar, durchgeführt. Für den erfindungsgemäß bevorzugten Fall, dass als Edukt eine olefinische Verbindung mit 12 C-Atomen eingesetzt wird, wird Stufe (a2) bevorzugt bei einem Druck ≤ 120 mbar, besonders bevorzugt ≤ 70 mbar, ganz besonders bevorzugt ≤ 60 mbar, durchgeführt. Erfindungsgemäß können dem Fachmann bekannte Destillationskolonnen eingesetzt werden, bevorzugt sind solche, die mindestens 20, bevorzugt mindestens 25, besonders bevorzugt mindestens 30 theoretische Trennstufen aufweisen. In einer weiteren bevorzugten Ausführungsform befinden sich 35 bis 55% der Trennstufen im Abtriebsteil der Destillationskolonne. Das Rücklaufverhältnis beträgt in der bevorzugten Ausführungsform, dass eine olefinische Verbindung mit 12 C-Atomen als Edukt eingesetzt wird, 1 bis 2, bevorzugt 1,2 bis 1,8. Für die anderen genannten Edukte kann das Rücklaufverhältnis durch den Fachmann angepasst werden.

Als Kopfprodukt dieser Destillation wird im Wesentlichen reines Edukt, d. h. mindestens ein cyclisches Olefin mit Z Cyclen und 7 bis 16 C-Atomen mit mindestens zwei C-C-Doppelbindungen, erhalten, welches in einer besonders bevorzugten Ausführungsform als Substrat in Stufe (a1) des erfindungsgemäßen Verfahrens zurückgeführt wird.

Das in der beschriebenen Destillation in Stufe (a2) erhaltene Sumpfprodukt entspricht im Wesentlichen der oben beschriebenen Zusammensetzung (A2).

In der bevorzugten Ausführungsform des erfindungsgemäßen Verfahrens, dass die Zusammensetzung (A1) auch mindestens eine cyclische Verbindung mit Z Cyclen und 7 bis 16 C-Atomen und mindestens zwei Ketogruppen enthält, wird in Stufe (a2) des erfindungsgemäßen Verfahrens eine Zusammensetzung (A2) erhalten, mindestens enthaltend
- die mindestens eine cyclische Verbindung mit Z Cyclen und 7 bis 16 C-Atomen mit einer Ketogruppe,
- mindestens eine cyclische Verbindung mit Z Cyclen und 7 bis 16 C-Atomen mit mindestens zwei Ketogruppen und
- mindestens eine Verbindung mit Z-1 Cyclen und 7 bis 16 C-Atomen mit mindestens einer Aldehydgruppe.

### Schritt (b):

Da die Zusammensetzung (A1) auch mindestens eine cyclische Verbindung mit Z Cyclen und 7 bis 16 C-Atomen und mindestens zwei Ketogruppen enthält, wird in Stufe (b) des erfindungsgemäßen Verfahrens eine Zusammensetzung (B) erhalten, mindestens enthaltend
- die mindestens eine cyclische Verbindung mit Z Cyclen und 7 bis 16 C-Atomen mit einer Ketogruppe,
- weniger als 0,5 Gew.-% der mindestens einen cyclischen Verbindung mit Z Cyclen und 7 bis 16 C-Atomen mit mindestens zwei Ketogruppen und
- weniger als 1,0 Gew.-% der mindestens einen Verbindung mit Z-1 Cyclen und 7 bis 16 C-Atomen mit mindestens einer Aldehydgruppe.

Die Mengenangaben beziehen sich jeweils auf die gesamte Zusammensetzung (B). Die vorliegende Erfindung besteht unter anderem darin, dass die Zusammensetzung (A2) aus Schritt (a2), destillativ behandelt wird, um die oben beschriebenen Zusammensetzungen (B) zu erhalten.

Dabei ist es erfindungswesentlich, dass die Zusammensetzung (B) weniger als 1,0 Gew.-%, bevorzugt weniger als 0,5 Gew.-%, besonders bevorzugt weniger als 0,2 Gew.-% der mindestens einer Verbindung mit Z-1 Cyclen und 7 bis 16 C-Atomen mit mindestens einer Aldehyd-Gruppe, insbesondere die genannten Dodecatrienal-Isomere enthält, jeweils bezogen auf die gesamte Zusammensetzung (B).

In einer besonders bevorzugten Ausführungsform enthält die Zusammensetzung (B) weniger als 0,3 Gew.-%, besonders bevorzugt weniger als 0,25 Gew.-% der mindestens einen cyclischen Verbindung mit Z Cyclen und 7 bis 16 C-Atomen mit mindestens zwei Ketogruppen, insbesondere die genannten Cyclododecendion-Isomere, und weniger als 1,0 Gew.-%, bevorzugt weniger als 0,5 Gew.-%, besonders bevorzugt weniger als 0,2 Gew.-% der mindestens einen Verbindung mit Z-1 Cyclen und 7 bis 16 C-Atomen mit mindestens einer Aldehyd-Gruppe, insbesondere die genannten Dodecatrienal-Isomere, jeweils bezogen auf die gesamte Zusammensetzung (B).

Es ist somit erfindungswesentlich, dass die mindestens eine Verbindung mit Z-1 Cyclen und 7 bis 16 C-Atomen mit mindestens einer Aldehyd-Gruppe in Stufe (b) des erfindungsgemäßen Verfahrens auf eine besonders geringe, oben genannte, Menge abgereichert wird. In einer besonders bevorzugten Ausführungsform des erfindungsgemäßen Verfahrens werden sowohl die mindestens eine cyclische Verbindung mit Z Cyclen und 7 bis 16 C-Atomen mit mindestens zwei Ketogruppen und die mindestens eine Verbindung mit Z-1 Cyclen und 7 bis 16 C-Atomen mit mindestens einer Aldehyd-Gruppe jeweils auf besonders geringe, oben genannte, Mengen abgereichert.

In einer bevorzugten Ausführungsform wird Stufe (b) des erfindungsgemäßen Verfahrens in wenigstens zwei Kolonnen durchgeführt. In einer besonders bevorzugten Ausführungsform wird Zusammensetzung (A2) aus Schritt (a2) in einem ersten Schritt in einer einfachen Destillationskolonne (T1) behandelt. Dabei erhält man bevorzugt einen Kopfstrom (K1), der im Wesentlichen aus mindestens einer Verbindung mit Z-1 Cyclen und 7 bis 16 C-Atomen mit mindestens einer Aldehydgruppe besteht, und gegebenenfalls maximal 35 Gew.-%, bevorzugt maximal 30 Gew.-%, besonders bevorzugt maximal 25 Gew.-% mindestens eine cyclische Verbindung mit Z Cyclen und 7 bis 16 C-Atomen mit mindestens zwei Ketogruppen enthält. Des Weiteren wird ein Sumpfstrom (S1) erhalten, der alle restlichen Komponenten enthalten kann.

Als Destillationskolonne (T1) können alle dem Fachmann als geeignet bekannten Kolonnen verwendet werden. In einer bevorzugten Ausführungsform enthält die Kolonne (T1) mindestens 10 theoretische Trennstufen, besonders bevorzugt mindestens 15 theoretische Trennstufen, ganz besonders bevorzugt mindestens 20 theoretische Trennstufen. Dabei ist es weiterhin bevorzugt, dass die meisten Trennstufen, beispielsweise mindestens 50% der Trennstufen, im Verstärkungsteil der Kolonne vorliegen. Die Destillation in der Destillationskolonne (T1) wird bevorzugt bei einem Druck unterhalb von Atmosphärendruck durchgeführt, beispielsweise, insbesondere für den bevorzugten Fall, dass als Edukt eine olefinische Verbindung mit 12 C-Atomen eingesetzt wird, bei einem Kopfdruck von weniger als 50 mbar, besonders bevorzugt weniger als 25 mbar. Für die anderen erfindungsgemäß einsetzbaren Verbindungen ist ein geeigneter Druck durch den Fachmann zu ermitteln. Die Destillation in der Destillationskolonne (T1) wird, für den bevorzugten Fall, dass als Edukt eine olefinische Verbindung mit 12 C-Atomen eingesetzt wird, bevorzugt bei einer Sumpftemperatur von 120 bis 220 °C, besonders bevorzugt 150 bis 200 °C, durchgeführt. Für die anderen erfindungsgemäß geeigneten Edukte kann die Destillationstemperatur, auch in Abhängigkeit vom eingestellten Druck, durch den Fachmann gewählt werden.

In einer weiteren bevorzugten Ausführungsform von Schritt (b) des erfindungsgemäßen Verfahrens wird der Sumpfstrom (S1) aus der ersten Destillationskolonne (T1) in wenigstens einer weiteren einfachen Destillationskolonne (T2) behandelt. In einer bevorzugten Ausführungsform wird dabei ein Kopfstrom (K2) erhalten, der im Wesentlichen mindestens eine cyclische Verbindung mit Z Cyclen und 7 bis 16 C-Atomen mit mindestens einer Ketogruppe enthält. Dieser Kopfstrom (K2) enthält in einer bevorzugten Ausführungsform im Wesentlichen keine Verbindung mit Z-1 Cyclen und 7 bis 16 C-Atomen mit mindestens einer Aldehydgruppe, d.h. weniger als 1,0 Gew.-%, in einer bevorzugten Ausführungsform höchstens 0,2 Gew.-%. In der zweiten Destillation in T2 des erfindungsgemäßen Verfahrens (Schritt (b)) wird des Weiteren ein Sumpfstrom (S2) erhalten, der mindestens eine cyclische Verbindung mit Z Cyclen und 7 bis 16 C-Atomen mit mindestens zwei Ketogruppen und andere Nebenprodukte der Oxidation gemäß Schritt (a1) enthält, jedoch maximal 40 Gew.-%, bevorzugt maximal 25 Gew.-% der mindestens einen cyclischen Verbindung mit Z Cyclen und 7 bis 16 C-Atomen mit einer Ketogruppe enthält.

Als Destillationskolonne (T2) kann jede Kolonne eingesetzt werden, die dem Fachmann als geeignet bekannt ist. In einer bevorzugten Ausführungsform weist die Kolonne mindestens 30 Trennstufen auf, besonders bevorzugt mindestens 35 Trennstufen. In einer weiteren bevorzugten Ausführungsform liegen die meisten Trennstufen im Abtriebsteil vor, besonders bevorzugt liegen mindestens 28 theoretische Trennstufen im Abtriebsteil vor. Die Destillation in Trennkolonne (T2) erfolgt bevorzugt bei einem Druck unterhalb von Atmosphärendruck, für den bevorzugten Fall, dass eine olefinische Verbindung mit 12 C-Atomen als Edukt eingesetzt wird, beispielsweise bei einem Kopfdruck ≤ 50 mbar, besonders bevorzugt ≤ 25 mbar. Die Destillation in der Destillationskolonne (T2) wird für den bevorzugten Fall, dass eine olefinische Verbindung mit 12 C-Atomen eingesetzt wird, bevorzugt bei einer Sumpftemperatur von 120 bis 220 °C, besonders bevorzugt 150 bis 200 °C, durchgeführt. Für die anderen erfindungsgemäß geeigneten Edukte kann die Temperatur, auch in Abhängigkeit vom eingestellten Druck, durch den Fachmann leicht eingestellt werden.

Erfindungsgemäß ist es auch möglich, die Destillationskolonnen (T1) und (T2) in umgekehrter Reihenfolge zu betreiben, das heißt in der erste Kolonne die mindestens eine cyclische Verbindung mit Z Cyclen und 7 bis 16 C-Atomen mit mindestens einer Ketogruppe und die mindestens eine Verbindung mit Z-1 Cyclen und 7 bis 16 C-Atomen mit mindestens einer Aldehydgruppe über Kopf zu trennen und in der zweite Kolonne die gereinigte mindestens eine cyclische Verbindung mit Z Cyclen und 7 bis 16 C-Atomen mit mindestens einer Ketogruppe über Sumpf zu trennen.

Ganz besonders bevorzugt wird anstelle der zwei Destillationskolonnen (T1) und (T2) in Schritt (b) des erfindungsgemäßen Verfahrens eine einzige Trennwandkolonne eingesetzt.

Erfindungsgemäß kann jede Trennwandkolonne in Schritt (b) des erfindungsgemäßen Verfahrens eingesetzt werden, die für den Fachmann für das vorliegende Trennproblem als geeignet erscheint.

In einer bevorzugten Ausführungsform wird eine kontinuierliche Trennwandkolonne eingesetzt, die bevorzugt über wenigstens drei Bereiche verfügt. Bevorzugt verfügt die Trennwandkolonne über einen unteren Bereich, der bevorzugt wenigstens 2, besonders bevorzugt wenigstens 4 theoretische Böden aufweist. Des Weiteren weist die bevorzugt eingesetzte Trennwandkolonne einen mittleren Teil auf, der bevorzugt wenigstens 15, besonders bevorzugt wenigstens 25, theoretische Böden aufweist. In einer weiteren bevorzugten Ausführungsform weist die eingesetzte Trennwandkolonne einen oberen Teil auf, der bevorzugt wenigstens 4, besonders bevorzugt wenigstens 7, theoretische Böden aufweist. Der mittlere Teil ist durch eine bevorzugt mittig angeordnete Trennwand in einen Eingangs- und einen Ausgangsbereich unterteilt.

In einer weiteren bevorzugten Ausführungsform ist die Trennwandkolonne mit einer geeigneten Packung bestückt. Geeignete Kolonnenpackungen sind dem Fachmann bekannt, beispielsweise aus J. F. Fair in "Handbook of Separation Process Technology", R. W. Rousseau (Hrsg.), (1987), John Wiley & Sons, Seiten 295 - 312.

Um die Temperatur bei der Destillation in der Trennwandkolonne möglichst niedrig halten zu können, wird bevorzugt bei einem Druck unterhalb von Atmosphärendruck gearbeitet, beispielsweise bei einem Druck innerhalb der Trennwandkolonne von weniger als 500 mbar, bevorzugt weniger als 200 mbar, insbesondere weniger als 100 mbar und ganz besonders bevorzugt weniger als 50 mbar. Der Druckunterschied zwischen Kolonnensumpf und Kolonnenkopf beträgt bevorzugt weniger als 50 mbar, besonders bevorzugt weniger als 30 mbar.

In der Trennwandkolonne wird die Destillation bevorzugt bei einer Sumpftemperatur von 150 bis 220 °C, besonders bevorzugt 160 bis 200 °C durchgeführt. Die Temperatur am Seitenabzug, an dem bevorzugt Zusammensetzung (B) entnommen wird, beträgt bevorzugt 110 bis 180 °C, besonders bevorzugt 130 bis 170 °C. Die Kopftemperatur der Trennwandkolonne beträgt bevorzugt 80 bis 150 °C, besonders bevorzugt 100 bis 135 °C. Diese Werte gelten insbesondere für den bevorzugten Fall, dass eine olefinische Verbindung mit 12 C-Atomen als Edukt eingesetzt wird, für die anderen erfindungsgemäß einsetzbaren Edukte können die Werte durch den Fachmann entsprechend angepasst werden.

Über Kopf der Trennwandkolonne werden bevorzugt leichtsiedende Komponenten der Zusammensetzung (A2) als Kopfstrom (K3) abgetrennt. In einer bevorzugten Ausführungsform enthält Kopfstrom (K3) wenigstens 30 Gew.-%, besonders bevorzugt wenigstens 50 Gew.-%, ganz besonders bevorzugt wenigstens 70 Gew.-%, mindestens eine Verbindung mit Z-1 Cyclen und 7 bis 16 C-Atomen und mindestens einer Aldehydgruppe.

Über Sumpf der Trennwandkolonne werden bevorzugt schwersiedende Komponenten der Zusammensetzung (A2) als Sumpfstrom (S2) abgetrennt. In einer bevorzugten Ausführungsform enthält Sumpfstrom (S2) mindestens eine cyclische Verbindung mit Z Cyclen und 7 bis 16 C-Atomen mit mindestens zwei Ketogruppen und andere Nebenprodukte der Oxidation gemäß Schritt (a1), jedoch maximal 40 Gew.-%, bevorzugt maximal 25 Gew.-% der mindestens einen cyclischen Verbindung mit Z Cyclen und 7 bis 16 C-Atomen mit einer Ketogruppe.

Über Seitenabzug der Trennwandkolonne wird Zusammensetzung (B) erhalten.

Nach der destillativen Behandlung gemäß Schritt (b) des erfindungsgemäßen Verfahrens wird eine Zusammensetzung (B) erhalten, die im Wesentlichen das gewünschte Produkt, die mindestens eine cyclische Verbindung mit Z Cyclen und 7 bis 16 C-Atomen mit einer Ketogruppe enthält.

Des Weiteren liegen in der erfindungsgemäßen Zusammensetzung (B) als Nebenprodukte mindestens eine cyclische Verbindung mit Z Cyclen und 7 bis 16 C-Atomen mit mindestens zwei Ketogruppen und mindestens eine Verbindung mit Z-1 Cyclen 7 bis 16 C-Atomen mit mindestens einer Aldehydgruppe in den oben angegebenen geringen Mengen vor.

Die Vorteile des erfindungsgemäßen Verfahrens, insbesondere von Schritt (b), liegen darin, dass durch die destillative Behandlung der Zusammensetzung (A2) eine Zusammensetzung (B) erhalten wird, die einen sehr geringen Gehalt an mindestens einer Verbindung mit Z-1 Cyclen und 7 bis 16 C-Atomen mit mindestens einer Aldehydgruppe aufweist. Liegt dieses Nebenprodukte der Oxidation gemäß Stufe (a1) in höheren Mengen als durch das erfindungsgemäße Verfahren möglich vor, so, wie es in den Verfahren des Standes der Technik üblich ist, ergeben sich im weiteren Verlauf des Verfahrens die folgenden Nachteile.

Wird die mindestens eine Verbindung mit Z-1 Cyclen und 7 bis 16 C-Atomen und mindestens einer Aldehydgruppe nicht durch die erfindungsgemäße Stufe (b) abgetrennt, so werden durch eine gegebenenfalls durchgeführte Hydrierung die entsprechenden gesättigten Aldehydgruppen bzw. Hydroxygruppen tragenden Verbindungen gebildet, insbesondere bevorzugt beispielsweise Dodecanal und/oder Dodecanol. Diese Nebenprodukte sind in dem bevorzugten Fall, dass das gewünschte Hauptprodukt Cyclododecanon ist, nur schwer durch übliche Verfahren abzutrennen, so dass es nicht möglich ist, ein hochreines Produkt zu erhalten.

Im erfindungsgemäß bevorzugten Fall, dass in der Oxidation gemäß Schritt (a1) des erfindungsgemäßen Verfahrens neben dem gewünschten Produkt u. a. auch mindestens eine cyclische Verbindung mit Z Cyclen und 7 bis 16 C-Atomen mit mindestens zwei Ketogruppen gebildet wird, können darüber hinaus die folgenden Nebenreaktionen stattfinden.

Unter dem Einfluss einer Base gehen die genannten Diketone sehr leicht eine intramolekulare Aldol-Reaktion ein. So wandelt sich unter basischen Bedingungen beispielsweise cis-Cyclododec-8-en-1,5-dion (XII), welches mit ca. 38% das Hauptisomer bei den in Schritt (a1) des erfindungsgemäßen Verfahrens bevorzugt gebildeten Diketonen sein kann, in (Z)-3,4,5,6,7,10-Hexahydro-2H-benzocycloocten-1-on (XIV) um. Bei einer gegebenenfalls durchgeführten anschließenden Hydrierung wird dieses in Decahydrobenzocycloocten-1-on (XV) überführt.

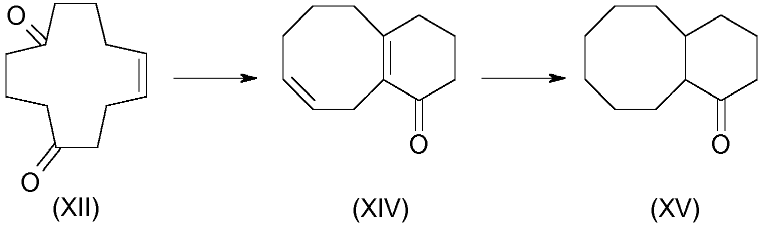

Decahydrobenzocycloocten-1-on (XV) und ähnliche bicyclische C₁₂-Ketone, die sich aus den anderen Cyclododecendionen bilden, weisen Siedepunkte auf, die nah an dem Siedepunkt von Cyclododecanon liegen, so dass es bei einer abschließenden destillativen Reinigung schwierig bzw. unmöglich ist, dieses gewünschte Produkt in der angestrebten hohen Reinheit zu erhalten. Auch die anderen erfindungsgemäß einsetzbaren Edukte führen zu entsprechenden Nebenprodukten, die destillativ nur schwierig abzutrennen sind.

Beispielsweise werden bei Einsatz von Cycloocta-1,5-dien (I) die folgenden Nebenprodukte erhalten:

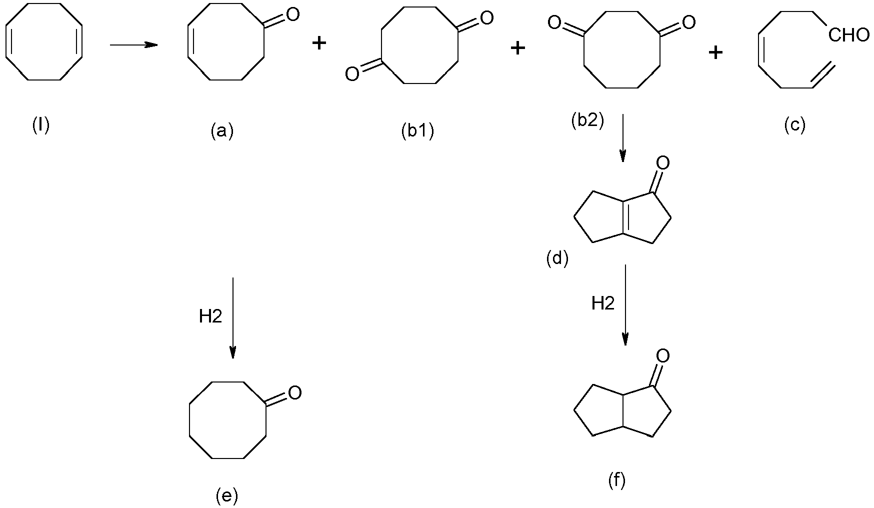

In dieser speziellen Ausführungsform des erfindungsgemäßen Verfahrens bildet sich in der Oxidation mit N₂O eine Mischung aus dem gewünschten ungesättigten Monoketon (a), aus den zwei Diketonen (b1) und (b2), welche der mindestens einen cyclischen Verbindung mit Z Cyclen und 7 bis 16 C-Atomen und zwei Ketogruppen entsprechen, und aus dem Aldehyd (c), welcher der mindestens einen Verbindung mit Z-1 Cyclen und 7 bis 16 C-Atomen mit einer Aldehydgruppe entspricht. Das Keton (b2) geht sehr leicht eine intramolekulare Aldolkondensation (z. T. schon alleine durch thermische Belastung) unter Bildung von (d) ein. Wenn die Diketone nicht abgetrennt werden, bevorzugt durch den erfindungsgemäßen Schritt (b), bildet sich (d) was anschließend zu (f) hydriert wird. Die Siedepunkte von (e), welches nach Hydrierung das gewünschte Produkt des gesamten Verfahrens ist, und (f) sind aber sehr ähnlich (e: 74°C und f: 72°C jeweils bei 12 Torr), was die Reinigung von (e) extrem erschwert.

Die aus dem Stand der Technik bekannten Verfahren, in denen die aus der Oxidationsreaktion erhaltenen Reaktionsmischungen enthaltend Diketone zunächst hydriert werden, weisen einen ähnlichen Nachteil auf. Durch Hydrierung erhält man aus den ungesättigten Diketonen, beispielsweise aus den oben genannten Cyclodecendionen (XII), die entsprechenden gesättigten Verbindungen, beispielsweise Cyclododecandione, insbesondere Cyclododecan-1,5-dion (XVI). Unter den Bedingungen einer gegebenenfalls durchgeführten thermischen Behandlung in Gegenwart eines Katalysators der Reaktionsmischung, beispielsweise wie in WO 2008/000754 beschrieben, wandelt sich beispielsweise Cyclododecan-1,5-dion (XVI), welches mit 50% das Hauptisomer bei den gebildeten Cyclododecandionen darstellt, in 3,4,5,6,7,8,9,10-Octahydro-(2H)-benzocycloocten-1-on (XVII) um.

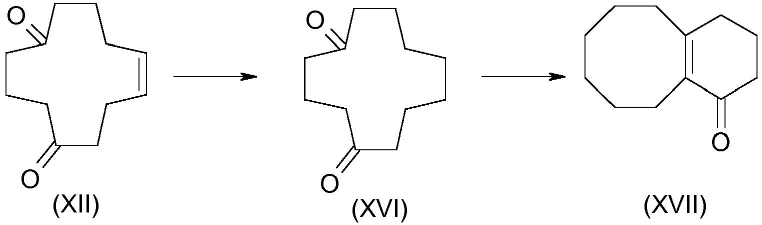

Verbindung (XVII) weist einen Siedepunkt auf, der ebenfalls sehr nahe an dem des bevorzugt gewünschten Wertproduktes Cyclododecanon liegt, so dass eine destillative Trennung schwierig bzw. unmöglich ist.

Durch Schritt (b) des erfindungsgemäßen Verfahrens ist es möglich, die genannten Nebenprodukte der Oxidation, beispielsweise ungesättigte Aldehyde und gegebenenfalls Diketone so umfassend aus der Reaktionsmischung abzutrennen, dass die beschriebenen, unerwünschten Nebenreaktionen nicht bzw. nur in geringem Maße stattfinden können, so dass die schwierig bzw. nicht abzutrennenden Verbindungen nicht bzw. nur in geringen Mengen gebildet werden können.

### Stufe c):

In einer weiteren bevorzugten Ausführungsform des erfindungemäßen Verfahrens schließt sich an Stufe (b) mindestens die folgende Stufe (c) an:
(c) Hydrierung der Zusammensetzung (B) in Gegenwart mindestens eines Katalysators, um eine Zusammensetzung (C) zu erhalten.

Unabhängig davon, welches Regioisomere der wenigstens einen cyclischen Verbindung mit Z Cyclen und 7 bis 16 C-Atomen mit einer Ketogruppe, beispielsweise Cyclododecadienon, oder welches Gemisch aus mindestens zwei regioisomeren cyclischen Verbindungen mit Z Cyclen und 7 bis 16 C-Atomen mit einer Ketogruppe aus der erfindungsgemäßen Umsetzung mit Distickstoffmonoxid gemäß Schritt (a) und aus der destillativen Behandlung gemäß Schritt (b) erhalten wird, enthalten diese Verbindungen durchweg wenigstens eine verbliebene, d. h. nicht oxidierte, Doppelbindung, welche im optionalen Schritt (c) des erfindungsgemäßen Verfahrens hydriert wird, um eine Zusammensetzung (C) enthaltend mindestens eine gesättigte cyclische Verbindung mit Z Cyclen und 7 bis 16 C-Atomen und einer Ketogruppe, zu erhalten.

Gemäß einer besonders bevorzugten Ausführungsform von Stufe (c) des erfindungsgemäßen Verfahrens wird Cyclododeca-4,8-dienon zu Cyclododecanon hydriert.
Demgemäß betrifft die vorliegende Erfindung auch ein wie oben beschriebenes Verfahren, wobei das aus der Umsetzung von Cyclododecatrien mit Distickstoffmonoxid gemäß Schritt (a1), der Behandlung gemäß Schritt (a2) und der destillativen Behandlung gemäß Schritt (b) erhaltene Cyclododecadienon in Schritt (c) unter Erhalt von Cyclododecanon hydriert wird.

Für die Hydrierung von Zusammensetzung (B), insbesondere bevorzugt von Cyclododeca-4,8-dienon, können sämtliche geeigneten Katalysatoren eingesetzt werden. Insbesondere sind mindestens ein homogener oder mindestens ein heterogener oder sowohl mindestens ein homogener und mindestens ein heterogener Katalysator einsetzbar.

Bevorzugt enthalten die einsetzbaren Katalysatoren mindestens ein Metall aus der 7., der 8., der 9., der 10. oder der 11. Nebengruppe des Periodensystems der Elemente. Weiter bevorzugt enthalten die erfindungsgemäß im optionalen Schritt (C) einsetzbaren Katalysatoren mindestens ein Element, ausgewählt aus der Gruppe bestehend aus Re, Fe, Ru, Co, Rh, Ir, Ni, Pd, Pt, Cu und Au. Insbesondere bevorzugt enthalten die erfindungsgemäß einsetzbaren Katalysatoren mindestens ein Element, ausgewählt aus der Gruppe bestehend aus Fe, Ni, Pd, Pt und Cu. Besonders bevorzugt enthalten die erfindungsgemäß eingesetzten Katalysatoren Pd.

Im optionalen Schritt (c) des erfindungsgemäßen Verfahrens bevorzugt eingesetzte homogene Katalysatoren enthalten mindestens ein Element der 8., 9. oder 10. Nebengruppe. Weiter bevorzugt sind homogene Katalysatoren, die Ru, Rh, Ir und/oder Ni enthalten. Beispielsweise sind hierbei etwa RhCl(TTP)₃ oder Ru₄H₄(CO)₁₂ zu nennen. Besonders bevorzugt sind solche homogenen Katalysatoren, die Ru enthalten. Beispielsweise werden homogene Katalysatoren eingesetzt, wie sie in der US 5,180, 870, US 5,321, 176, US 5,177, 278, US 3,804, 914, US 5,210, 349 US 5, 128, 296, US B 316,917 und in D. R. Fahey in J. Org. Chem. 38 (1973) S. 80-87 beschrieben sind, deren diesbezügliche Offenbarung in den Kontext der vorliegenden Anmeldung vollumfänglich einbezogen wird. Solche Katalysatoren sind etwa (TPP)₂(CO)₃Ru, [RU(CO)₄]₃, (TPP)₂Ru(CO)₂Cl₂, (TPP)₃(CO)RuH₂, (TPP)₂(CO)₂RuH₂, (TPP)₂(CO)₂RuClH oder (TPP)₃(CO)RuCl.

Insbesondere bevorzugt wird im optionalen Schritt (c) des erfindungsgemäßen Verfahrens mindestens ein heterogener Katalysator eingesetzt, wobei mindestens eines der oben stehend genannten Metalle als Metall als solches, als Raney-Katalysator und/oder aufgebracht auf einen üblichen Träger eingesetzt werden kann. Bevorzugte Trägermaterialien sind etwa Aktivkohlen oder Oxide wie beispielsweise Aluminiumoxide, Siliciumoxide, Titanoxide oder Zirkonoxide. Ebenso sind unter anderem als Trägermaterialien Bentonite zu nennen. Werden zwei oder mehr Metalle eingesetzt, so können diese separat oder als Legierung vorliegen. Hierbei ist es möglich, mindestens ein Metall als solches und mindestens ein anderes Metall als Raney-Katalysator oder mindestens ein Metall als solches und mindestens ein anderes Metall, aufgebracht auf mindestens einen Träger, oder mindestens ein Metall als Raney-Katalysator und mindestens ein anderes Metall, aufgebracht auf mindestens einen Träger, oder mindestens ein Metall als solches und mindestens ein anderes Metall als Raney-Katalysator und mindestens ein anderes Metall, aufgebracht auf mindestens einen Träger, einzusetzen.

Die im optionalen Schritt (c) des erfindungsgemäßen Verfahrens eingesetzten Katalysatoren können beispielsweise auch so genannte Fällungskatalysatoren sein. Solche Katalysatoren können hergestellt werden, indem man ihre katalytisch aktiven Komponenten aus deren Salzlösungen, insbesondere aus den Lösungen von deren Nitraten und/oder Acetaten, beispielsweise durch Zugabe von Lösungen von Alkalimetall- und/oder Erdalkalimetallhydroxid-und/oder Carbonatlösungen, beispielsweise schwerlöslichen Hydroxiden, Oxidhydraten, basischen Salze oder Carbonaten ausfällt, die erhaltenen Niederschläge anschließend trocknet und diese dann durch Calcinierung bei im Allgemeinen 300 bis 700 °C, insbesondere 400 bis 600 °C in die entsprechenden Oxide, Mischoxide und/oder gemischtvalentigen Oxide umwandelt, die durch eine Behandlung mit Wasserstoff oder mit Wasserstoff enthaltenden Gasen im Bereich von im Allgemeinen 50 bis 700 °C, insbesondere 100 bis 400 °C zu den betreffenden Metallen und/oder oxidischen Verbindungen niederer Oxidationsstufe reduziert und in die eigentliche katalytisch aktive Form überführt werden. Dabei wird in der Regel so lange reduziert, bis kein Wasser mehr gebildet wird. Bei der Herstellung von Fällungskatalysatoren, die ein Trägermaterial enthalten, kann die Fällung der katalytisch aktiven Komponenten in Gegenwart des betreffenden Trägermaterials erfolgen. Die katalytisch aktiven Komponenten können vorteilhaft gleichzeitig mit dem Trägermaterial aus den betreffenden Salzlösungen gefällt werden.

Bevorzugt werden im optionalen Schritt (c) des erfindungsgemäßen Verfahrens Hydrierkatalysatoren eingesetzt, welche die die Hydrierung katalysierenden Metalle oder Metallverbindungen auf einem Trägermaterial abgeschieden enthalten.

Außer den oben genannten Fällungskatalysatoren, die außer den katalytisch aktiven Komponenten noch zusätzlich ein Trägermaterial enthalten, eignen sich für das erfindungsgemäße Verfahren im Allgemeinen solche Trägermaterialien, bei denen die katalytisch hydrierend wirkende Komponente beispielsweise durch Imprägnierung auf ein Trägermaterial aufgebracht worden sind.

Die Art des Aufbringens des katalytisch aktiven Metalls auf den Träger ist in der Regel nicht kritisch und kann auf verschiedenerlei Art und Weise bewerkstelligt werden. Die katalytisch aktiven Metalle können auf diesen Trägermaterialien beispielsweise durch Tränkung mit Lösungen oder Suspensionen der Salze oder Oxide der betreffenden Elemente, Trocknung und anschließender Reduktion der Metallverbindungen zu den betreffenden Metallen oder Verbindungen niederer Oxidationsstufe mittels eines Reduktionsmittels, vorzugsweise mit Wasserstoff oder komplexen Hydriden, aufgebracht werden. Eine andere Möglichkeit zur Aufbringung der katalytisch aktiven Metalle auf diese Träger besteht darin, die Träger mit Lösungen thermisch leicht zersetzbarer Salze, beispielsweise mit Nitraten oder thermisch leicht zersetzbaren Komplexverbindungen, beispielsweise Carbonyl- oder Hydrido-Komplexen der katalytisch aktiven Metalle, zu imprägnieren und den so getränkten Träger zur thermischen Zersetzung der adsorbierten Metallverbindungen auf Temperaturen im Bereich von 300 bis 600 °C zu erhitzen. Diese thermische Zersetzung wird vorzugsweise unter einer Schutzgasatmosphäre vorgenommen. Geeignete Schutzgase sind beispielsweise Stickstoff, Kohlendioxid, Wasserstoff oder die Edelgase. Weiterhin können die katalytisch aktiven Metalle auf dem Katalysatorträger durch Aufdampfen oder durch Flammspritzen abgeschieden werden. Der Gehalt dieser Trägerkatalysatoren an den katalytisch aktiven Metallen ist prinzipiell für das Gelingen des erfindungsgemäßen Verfahrens nicht kritisch. Im Allgemeinen führen höhere Gehalte an katalytisch aktiven Metallen dieser Trägerkatalysatoren zu höheren Raum-Zeit-Umsätzen als niedrigere Gehalte. Im Allgemeinen werden Trägerkatalysatoren verwendet, deren Gehalt an katalytisch aktiven Metallen im Bereich von 0,1 bis 90 Gew.-%, vorzugsweise im Bereich von 0,5 bis 40 Gew.-% bezogen auf das Gesamtgewicht des Katalysators, liegt. Da sich diese Gehaltsangaben auf den gesamten Katalysator inklusive Trägermaterial beziehen, die unterschiedlichen Trägermaterialien jedoch sehr unterschiedliche spezifische Gewichte und spezifische Oberflächen haben, ist es auch denkbar, dass diese Angaben auch unter- oder überschritten werden können, ohne dass sich dies nachteilig auf das Ergebnis des erfindungsgemäßen Verfahrens auswirkt. Selbstverständlich können auch mehrere der katalytisch aktiven Metalle auf dem jeweiligen Trägermaterial aufgebracht sein. Weiterhin können die katalytisch aktiven Metalle beispielsweise nach dem Verfahren von DE-OS 25 19 817 oder EP 0 285 420 A1 auf den Träger aufgebracht werden.

Sowohl die Aktivierung der Fällungskatalysatoren als auch der Trägerkatalysatoren kann auch in situ zu Beginn der Reaktion durch den anwesenden Wasserstoff erfolgen. Bevorzugt werden diese Katalysatoren vor ihrer Verwendung separat aktiviert.

Als Trägermaterialien können im Allgemeinen die Oxide des Aluminiums und Titans, Zirkoniumdioxid, Siliciumdioxid, Tonerden wie beispielsweise Montmorillonite, Silikate wie beispielsweise Magnesium- oder Aluminiumsilikate, Zeolithe wie beispielsweise der Strukturtypen ZSM-5 oder ZSM-10, oder Aktivkohle verwendet werden. Bevorzugte Trägermaterialien sind Aluminiumoxide, Titandioxide, Siliciumdioxid, Zirkoniumdioxid und Aktivkohle. Selbstverständlich können auch Mischungen verschiedener Trägermaterialien als Träger für im optionalen Schritt (c) des erfindungsgemäßen Verfahrens verwendbare Katalysatoren dienen.

Der mindestens eine heterogene Katalysator kann im optionalen Schritt (c) des erfindungsgemäßen Verfahrens beispielsweise als Suspensionskatalysator und/oder als Festbettkatalysator eingesetzt werden.

Wird beispielsweise im Rahmen des erfindungsgemäßen Verfahrens die optionale Hydrierung gemäß Schritt (c) mit mindestens einem Suspensionskatalysator durchgeführt, so wird bevorzugt in mindestens einem Rührreaktor, oder in mindestens einer Blasensäule oder in mindestens einer gepackten Blasensäule oder in einer Kombination aus zwei oder mehr gleichen oder unterschiedlichen Reaktoren hydriert.

Der Begriff "unterschiedliche Reaktoren" bezeichnet vorliegend sowohl unterschiedliche Reaktortypen als auch Reaktoren der gleichen Art, die sich beispielsweise durch ihre Geometrie wie beispielsweise ihrem Volumen und/oder ihrem Querschnitt und/oder durch die Hydrierbedingungen in den Reaktoren unterscheiden.

Wird beispielsweise im Rahmen des optionalen Schrittes (c) des erfindungsgemäßen Verfahrens die Hydrierung mit mindestens einem fest angeordneten Katalysator durchgeführt, so wird bevorzugt mindestens ein Rohrreaktor wie beispielsweise mindestens ein Schachtreaktor und/oder mindestens ein Rohrbündelreaktor verwendet, wobei ein einzelner Reaktor in Sumpf- oder Rieselfahrweise betrieben werden kann. Bei Verwendung von zwei oder mehr Reaktoren kann mindestens einer in Sumpffahrweise und mindestens einer in Rieselfahrweise betrieben werden.

Gemäß einer bevorzugten Ausführungsform des optionalen Schrittes (c) des erfindungsgemäßen Verfahrens wird der für die Hydrierung eingesetzte mindestens eine Katalysator aus dem Produktgemisch der Hydrierung abgetrennt. Diese Abtrennung kann in Abhängigkeit vom eingesetzten Katalysator gemäß jeder geeigneten Verfahrensführung erfolgen.

Wird als Katalysator bei der Hydrierung beispielsweise ein heterogener Katalysator als Suspensionskatalysator eingesetzt, so wird dieser im Rahmen der vorliegenden Erfindung bevorzugt durch mindestens einen Filtrationsschritt abgetrennt. Der derart abgetrennte Katalysator kann in die Hydrierung rückgeführt werden oder mindestens einem beliebigen anderen Verfahren zugeführt werden. Ebenso ist es möglich, den Katalysator aufzuarbeiten, um beispielsweise das in dem Katalysator enthaltene Metall zurück zu gewinnen.

Wird als Katalysator bei der Hydrierung beispielsweise ein homogener Katalysator eingesetzt, so wird dieser im Rahmen der vorliegenden Erfindung bevorzugt durch mindestens einen Destillationsschritt abgetrennt. Im Rahmen dieser Destillation können eine oder zwei oder mehr Destillationskolonnen verwendet werden. Der derart abgetrennte Katalysator kann in die Hydrierung rückgeführt werden oder mindestens einem beliebigen anderen Verfahren zugeführt werden. Ebenso ist es möglich, den Katalysator aufzuarbeiten, um beispielsweise das in dem Katalysator enthaltene Metall zurück zu gewinnen.

Vor dem Einsatz in einem beliebigen Verfahren wie beispielsweise vor der Rückführung in das erfindungsgemäße Verfahren kann sowohl der mindestens eine homogene als auch der mindestens eine heterogene Katalysator, sollte dies erforderlich sein, durch mindestens ein geeignetes Verfahren regeneriert werden.

Die Wärmeabfuhr kann bei dem erfindungsgemäß verwendeten Reaktor intern beispielsweise über Kühlschlangen und/oder extern beispielsweise über mindestens einen Wärmetauscher abgeführt werden. Wird beispielsweise bevorzugt mindestens ein Rohrreaktor zur Hydrierung eingesetzt, so wird bevorzugt die Reaktion über äußeren Umlauf gefahren, in dem die Wärmeabführung integriert ist.

Wird gemäß einer bevorzugten Ausführungsform des erfindungsgemäßen Verfahrens die Hydrierung kontinuierlich durchgeführt, werden weiter bevorzugt mindestens zwei Reaktoren, weiter bevorzugt mindestens zwei Rohrreaktoren, weiter bevorzugt mindestens zwei seriell gekoppelte Rohrreaktoren und insbesondere bevorzugt zwei seriell gekoppelte Rohrreaktoren eingesetzt. Die Hydrierbedingungen in den eingesetzten Reaktoren können jeweils gleich oder unterschiedlich sein und liegen jeweils in den oben beschriebenen Bereichen.

Wird die Hydrierung an mindestens einem suspendierten Katalysator durchgeführt, liegt die Verweilzeit im Allgemeinen im Bereich von 0,5 bis 50 h, bevorzugt im Bereich von 1 bis 30 h und besonders bevorzugt im Bereich von 1,5 bis 25 h. Dabei ist es unerheblich, ob erfindungsgemäß ein Reaktor oder mindestens 2 in Serie geschaltete Reaktoren eingesetzt werden. Für sämtliche dieser Ausführungsformen liegt die Gesamtverweilzeit in den oben angegebenen Bereichen.

Wird im Rahmen des erfindungsgemäßen Verfahrens die Hydrierung in kontinuierlicher Fahrweise an mindestens einem fest angeordneten Katalysator durchgeführt, so liegt die Verweilzeit im Allgemeinen im Bereich von 0,1 bis 20 h, bevorzugt im Bereich von 0,2 bis 15 h und besonders bevorzugt im Bereich von 0,3 bis 10 h. Dabei ist es unerheblich, ob erfindungsgemäß ein Reaktor oder mindestens 2 in Serie geschaltete Reaktoren eingesetzt werden. Für sämtliche dieser Ausführungsformen liegt die Gesamtverweilzeit in den oben angegebenen Bereichen.

Das Gemisch, das aus dem ersten Rohrreaktor erhalten wird, enthält das gewünschte Produkt, beispielsweise Cyclododecanon, in einem Anteil, bevorzugt im Bereich von 50 bis 99,9 Gew. -% und besonders bevorzugt im Bereich von 70 bis 99,5 Gew. -%. Dieses Gemisch wird, gegebenenfalls nach mindestens einer geeigneten Zwischenbehandlung, dem zweiten Rohrreaktor zugeführt. Das Gemisch, das aus dem zweiten Rohrreaktor erhalten wird, enthält Cyclododecanon, in einem Anteil bevorzugt im Bereich von mindestens 99,5 %, weiter bevorzugt im Bereich von 99,9 % und insbesondere von 99,99 Gew. -%., besonders bevorzugt im Bereich von mindestens 99,9 % und insbesondere bevorzugt von mindestens 99,99 Gew. -%.

Der Wasserstoffdruck liegt bei der erfindungsgemäßen Hydrierung in dem optionalen Schritt (c) im Allgemeinen im Bereich von 1 bis 325 bar, bevorzugt im Bereich von 1,5 bis 200 bar, weiter bevorzugt im Bereich von 2 bis 100 bar und insbesondere bevorzugt im Bereich von 2,5 bis 50 bar.

Die Hydriertemperatur liegt im Allgemeinen im Bereich von 0 bis 250 °C, bevorzugt im Bereich von 20 bis 200 °C, beispielsweise im Bereich von 30 bis 180 °C, weiter bevorzugt im Bereich von 30 bis 150 °C, besonders bevorzugt im Bereich von 40 bis 170 °C und insbesondere bevorzugt im Bereich von 40 bis 140 °C.

Demgemäß betrifft die vorliegende Erfindung auch ein wie oben beschriebenes Verfahren, das dadurch gekennzeichnet ist, dass die Hydrierung in Schritt (c) in Anwesenheit eines Hydrierkatalysators, bevorzugt eines heterogenen Hydrierkatalysators, bei einer Temperatur im Bereich von 0 bis 250 °C und einem Druck im Bereich von 1 bis 325 bar durchgeführt wird.

Im Rahmen der erfindungsgemäßen Hydrierung kann mindestens ein geeignetes Lösungsmittel oder Verdünnungsmittel eingesetzt werden. Als solche sind unter anderem Cyclododecanon oder Cyclododecan sowie grundsätzlich sämtliche Lösungs- und Verdünnungsmittel zu nennen, die unter den Hydrierbedingungen nicht hydriert oder anderweitig umgesetzt werden.

Gemäß einer bevorzugten Ausführungsform des erfindungsgemäßen Verfahrens wird die Hydrierung ohne Zusatz eines Lösungs- oder Verdünnungsmittels durchgeführt.
Aus der erfindungsgemäßen Hydrierung wird im Allgemeinen ein Gemisch erhalten, das neben der mindestens einen cyclischen Verbindung mit Z Cyclen und 7 bis 16 C-Atomen und einer Ketogruppe, insbesondere Cyclododecanon, die oben genannten Nebenprodukte in den genannten sehr geringen Mengen aufweist.

Das erfindungsgemäße Verfahren zur Herstellung von mindestens einer cyclischen Verbindung mit Z Cyclen und 7 bis 16 C-Atomen mit einer Ketogruppe, beispielsweise Cyclododecanon, bietet unter anderem den Vorteil, dass dieses Keton in wenigen Schritten und gleichzeitig mit hoher Selektivität erhalten werden. Ein weiterer erheblicher Vorteil ist die Tatsache, dass als ein Edukt für das erfindungsgemäße Verfahren Distickstoffmonoxid enthaltende Abgase aus bevorzugt industriellen Anlagen eingesetzt werden können, die einerseits ohne großen Aufwand verfügbar sind, die andererseits die Integration des erfindungsgemäßen Verfahrens in einen bestehenden Anlagenverbund ermöglichen, wodurch der Transportweg für das Edukt minimal gehalten werden kann, und die weiterhin, als potentielle Klimagase, nicht einer besonderen Behandlung zur Entsorgung zugeführt werden müssen, sondern direkt in ein Wertprodukt fließen.

Die vorliegende Erfindung betrifft auch das erfindungsgemäße Verfahren, wobei sich an Stufe (b) mindestens die folgende Stufe (d) anschließt:
(d) Behandeln der Zusammensetzung (B) aus Stufe (b) mit mindestens einer Base, um eine Zusammensetzung (D) zu erhalten.

Erfindungsgemäß wird die Zusammensetzung (B) in der optionalen Stufe (d) mit mindestens einer Base behandelt. Dabei sind die Verfahrensbedingungen der Behandlung in weiten Bereichen variierbar, solange gewährleistet ist, dass die Konzentration mindestens einer störenden Nebenkomponente, insbesondere mindestens eines Aldehyds, beispielsweise einer Verbindung mit Z Cyclen und 7 bis 16 C-Atomen mit mindestens einer Aldehydgruppe, die in Stufe (a1) als Nebenprodukt gebildet werden kann, verringert wird.

Erfindungsgemäß wird dabei bevorzugt die Menge der mindestens einen Verbindung mit Z-1 Cyclen und 7 bis 16 C-Atomen mit mindestens einer Aldehydgruppe weiter verringert. Dabei werden erfindungsgemäß nach Schritt (b) gegebenenfalls noch vorhandene Verbindungen mit Z-1 Cyclen und 7 bis 16 C-Atomen mit mindestens einer Aldehydgruppe vorzugsweise zu mehr als 90% abgebaut, insbesondere zu mehr als 95% und besonders bevorzugt zu 99,99%.

In der Oxidation in Schritt (a1) gegebenenfalls gebildete Verbindungen mit Z Cyclen mit 7 bis 16 C-Atomen und mindestens einer Aldehydgruppe, d. h. cyclische Aldehyde die durch Ringkontraktion entstehen, werden vorzugsweise zu bis zu 30% abgebaut, insbesondere zu bis zu 35% und besonders bevorzugt zu bis zu 40%. Erfindungsgemäß wird die mindestens eine cyclische Verbindung mit Z Cyclen und 7 bis 16 C-Atomen und einer Ketogruppe nur zu 0,5 bis 2,0%, vorzugsweise zu 0,75 bis 1,75%, insbesondere zu 1,0 bis 1,5 % abgebaut.

In erster Näherung wird pro Mol Verbindung mit einer Aldehydgruppe ein Mol Wertprodukt, d. h. Verbindung mit Z Cyclen und 7 bis 16 C-Atomen und einer Ketogruppe, abgebaut.
Erfindungsgemäß erfolgt das Behandeln mit mindestens einer Base gemäß Stufe (d) vorzugsweise für einen Zeitraum von 1 Minute bis 10 Stunden, insbesondere 5 Minuten bis 5 Stunden, besonders bevorzugt 10 bis 60 Minuten, weiter bevorzugt 20 bis 50 Minuten.

Dabei kann das Behandeln im Rahmen des erfindungsgemäßen Verfahrens insbesondere bei einer Temperatur von 100 bis 250 °C erfolgen, bevorzugt 110 bis 220 °C, besonders bevorzugt 120 bis 200 °C, weiter bevorzugt 150 bis 190 °C.

Daher betrifft die vorliegende Erfindung gemäß einer weiteren Ausführungsform auch ein wie zuvor beschriebenes Verfahren zur Herstellung einer cyclischen Verbindung mit Z Cyclen und 7 bis 16 C-Atomen mit einer Ketogruppe, wobei die Behandlung gemäß Stufe (d) bei einer Temperatur von 100 bis 250 °C und für einen Zeitraum von 1 Minute bis 10 Stunden erfolgt.

Für die Behandlung mit der Base sind alle möglichen Reaktortypen geeignet. Für eine kontinuierliche Reaktionsführung werden bevorzugt Reaktoren mit einer Rohrcharakteristik, wie z.B. Rohrreaktoren, Rührkesselkaskaden oder vergleichbare Reaktoren eingesetzt. Für eine diskontinuierliche Reaktionsführung (Batch-Verfahren) sind einfache Rührkessel gut geeignet. Vorzugsweise läuft die Reaktion im Wesentlichen homogen in der Flüssigphase ab.

Vorzugsweise umfasst die Behandlung gemäß Stufe (d) zwei Teilschritte (d1) und (d2), wobei gemäß Schritt (d1) die Zusammensetzung (B) mit mindestens einer Base behandelt wird und gemäß Schritt (d2) die Base abgetrennt wird.

Daher betrifft die vorliegende Erfindung gemäß einer weiteren Ausführungsform auch ein wie zuvor beschriebenes Verfahren zur Herstellung einer cyclischen Verbindung mit Z Cyclen und 7 bis 16 C-Atomen mit einer Ketogruppe, wobei die Stufe (d) die Teilschritte (d1) und (d2) umfasst:
(d1) Behandeln der Zusammensetzung (B) mit mindestens einer Base,
(d2) Abtrennung der Base.

Die Abtrennung der Base gemäß (d2) kann gemäß allen üblichen Verfahren erfolgen, beispielsweise durch Destillation. Insbesondere wenn als Base NaOH oder KOH eingesetzt werden, erfolgt die Abtrennung bevorzugt durch Verdampfung, zum Beispiel in Form eines Fallfilmverdampfers, eines *wiped film evaporators* oder Wendelrohrverdampfers, oder durch Extraktion der Base, beispielsweise mit Wasser.

Im Rahmen der vorliegenden Erfindung können prinzipiell alle geeigneten Basen eingesetzt werden. Vorzugsweise werden organische oder anorganische Basen eingesetzt, deren konjugierte Säure einen pKₐ in Wasser von >9 haben. Bevorzugt sind im Rahmen der vorliegenden Erfindung beispielsweise Trialkylamine, Alkali- oder Erdalkalialkoholate und Tetraalkylammonium-, Alkali- oder Erdalkalihydroxide. Ganz besonders bevorzugt sind Natrium- und Kaliumhydroxid.

Die Base kann erfindungsgemäß entweder als reine Substanz oder als Lösung eingesetzt werden. Flüssige Basen werden bevorzugt ohne den Zusatz eines Lösungsmittels eingesetzt. Feste Basen werden bevorzugt als Lösung eingesetzt. Als Lösungsmittel wird bevorzugt die konjugierte Säure eingesetzt. Die besonders bevorzugten Basen NaOH und KOH werden bevorzugt als konzentrierte wässrige Lösung eingesetzt. Vorzugsweise weist eine als Lösung eingesetzte Base eine Konzentration von mindestens 25 Gew.-%, insbesondere von mindestens 40 Gew.-%, besonders bevorzugt von etwa 50 Gew.-% auf.

Die Menge der gemäß Stufe (d) eingesetzten Base kann in weiten Bereichen variiert werden. Es können zwischen 0,01 und 5 mol Base/mol Aldehyd eingesetzt werden. Bevorzugt werden zwischen 0,05 und 2 mol Base/mol Aldehyd eingesetzt. Besonders bevorzugt zwischen 0,1 und 1 mol Base/mol Aldehyd im zu behandelnden Gemisch, eingesetzt. Unter Aldehyd wird an dieser Stelle die Summe alle enthaltenen Aldehyde verstanden.

Das Behandeln mit der Base wird im Temperaturbereich zwischen 100 und 250 °C durchgeführt. Bevorzugt wird die Umsetzung zwischen 110 und 220 °C durchgeführt. Besonders bevorzugt wird die Umsetzung zwischen 150 und 190 °C durchgeführt. Die Dauer der Behandlung wird durch die gewählte Temperatur, Basenart und -menge und durch den gewünschten Abreicherungsgrad für die Aldehyde bestimmt. Dabei werden die Bedingungen vorzugsweise so gewählt, dass die Dauer des Behandelns zwischen 1 Minute und 10 Stunden liegt, beispielsweise zwischen 10 Minuten und 5 Stunden, bevorzugt zwischen 20 Minuten und 2 Stunden, insbesondere zwischen 30 Minuten und 1,5 Stunden, besonders bevorzugt zwischen 40 Minuten und 1 Stunde.

Gemäß einer bevorzugten Ausführungsform wird das Behandeln mit der Base bei einer Temperatur von 160 bis 185 °C für eine Zeit von 30 bis 40 Minuten durchgeführt. Vorzugsweise wird das Behandeln mit 0,1 bis 0,15 Gew.-% Natriumhydroxid, bezogen auf die gesamte Zusammensetzung, durchgeführt. Gemäß einer besonders bevorzugten Ausführungsform wird das Behandeln mit der Base bei einer Temperatur von 160 bis 185 °C für eine Zeit von 30 bis 40 Minuten mit 0,1 bis 0,15 Gew.-% Natriumhydroxid, bezogen auf die gesamte Zusammensetzung, durchgeführt.

Nach dem optionalen Schritt (d) des erfindungsgemäßen Verfahrens wird eine Zusammensetzung (D) erhalten. Diese Zusammensetzung (D) enthält bevorzugt mindestens 85 Gew.-% der cyclischen Verbindung mit Z Cyclen und 7 bis 16 C-Atomen und einer Ketogruppe, besonders bevorzugt mindestens 95 Gew.-% der cyclischen Verbindung mit Z Cyclen und 7 bis 16 C-Atomen und einer Ketogruppe.

Nach Schritt (d) des erfindungsgemäßen Verfahrens schließt sich ein Hydrierschritt (c') an.

Die vorliegende Erfindung betrifft daher auch das erfindungsgemäße Verfahren, wobei sich an Stufe (d) mindestens die folgende Stufe (c') anschließt:
(c') Hydrierung der Zusammensetzung (D) in Gegenwart mindestens eines Katalysators, um eine Zusammensetzung (C') zu erhalten.

Dieser Hydrierschritt wird im Allgemeinen analog zu dem oben beschriebenen optionalen Schritt (c) durchgeführt. Nach dem optionalen Schritt (c') wird eine Zusammensetzung (C') erhalten. Diese enthält in einer bevorzugten Ausführungsform mindestens 90 Gew.-% einer gesättigten cyclischen Verbindung mit Z Cyclen und 7 bis 16 C-Atomen mit einer Ketogruppe, besonders bevorzugt mindestens 95 Gew.-% einer gesättigten cyclischen Verbindung mit Z Cyclen und 7 bis 16 C-Atomen mit einer Ketogruppe. Bezüglich den Verfahrensparametern wie beispielsweise Katalysator, Druck, Temperatur, Stoffmengen etc. gilt für die erfindungsgemäße Stufe (c') das bezüglich Stufe (c) Gesagte.

In einer weiteren bevorzugten Ausführungsform des erfindungsgemäßen Verfahrens schließt sich an den Hydrierschritt (c) oder den optionalen Hydrierschritt (c') der folgende Schritt (e) an:
(e) Reinigen der Zusammensetzung (C) aus Schritt (c) oder (C') aus Schritt (c'), mindestens umfassend die Schritte
   (e1) thermisches Behandeln der Zusammensetzung (C) oder (C') mit mindestens einer Säure, oder mindestens einem Katalysator, der mindestens ein Übergangsmetall enthält,
   (e2) weiteres Aufreinigen durch ein Verfahren ausgewählt aus der Gruppe bestehend aus Destillation, Extraktion und Kristallisation.

Erfindungsgemäß kann die Zusammensetzung (C) oder (C') direkt in die Stufe (e) eingesetzt werden. Es ist jedoch auch möglich, dass die Zusammensetzung (C) oder (C') vor Stufe (e) einer Zwischenbehandlung unterworfen wird. In einer bevorzugten Ausführungsform wird Schritt (e) direkt im Anschluss an Schritt (c) durchgeführt, so dass die Schritte (d) und (c') nicht durchgeführt werden.

Die optionale Stufe (e) des erfindungsgemäßen Verfahrens umfasst Stufen (e1) und (e2). Gemäß Stufe (e1) wird die Zusammensetzung (C) oder (C') mit einer Säure oder mit einem Katalysator, der mindestens ein Übergangsmetall enthält, thermisch behandelt.
Die Behandlung mit Säure oder mit einem Katalysator, der mindestens ein Übergangsmetall enthält, erfolgt vorzugsweise bei Temperaturen von 30 bis 350 °C, beispielsweise 60 bis 350 °C, insbesondere von 100 bis 270 °C, besonders bevorzugt von 130 bis 260 °C.

Daher betrifft die vorliegende Erfindung gemäß einer weiteren Ausführungsform auch ein wie zuvor beschriebenes Verfahren zur Herstellung einer cyclischen Verbindung mit Z Cyclen und 7 bis 16 C-Atomen und einer Ketogruppe, wobei die Behandlung gemäß Schritt (e1) bei einer Temperatur von 60 bis 350 °C durchgeführt wird.

Es wurde überraschend gefunden, dass bei der Behandlung von Zusammensetzungen enthaltend mindestens eine cyclische Verbindung mit Z Cyclen und 7 bis 16 C-Atomen mit einer Ketogruppe mit Säuren oder mit einem Katalysator, der mindestens ein Übergangsmetall enthält, bei einer anschließenden weiteren Aufreinigung, beispielsweise durch Destillation, Extraktion und/oder durch Kristallisation cyclische Ketone in hohen Ausbeuten in Reinheiten von über 99,5% erhalten werden können. Dabei wird das cyclische Keton selbst nicht oder nur sehr unwesentlich angegriffen. Erfindungsgemäß handelt es sich bei den abgetrennten Verbindungen insbesondere um Alkohole, Aldehyde und Epoxide.

Bezogen auf das in der Zusammensetzung enthaltene cyclische Keton gehen erfindungsgemäß weniger als 10% des Ketons verloren, bevorzugt weniger als 5%, insbesondere weniger als 3%. Dabei kann das Behandeln gemäß Stufe (e1) sowohl in der Gasphase als auch in der Flüssigphase erfolgen. Der Druck ist dabei in weiten Bereichen einstellbar. Er kann beispielsweise zwischen 0,001 und 300 bar liegen, bevorzugt zwischen 0,01 und 200 bar, besonders bevorzugt zwischen 0,1 und 100 bar. Bevorzugt ist erfindungsgemäß ein Druck, bei dem gegebenenfalls entstehende Leichtsieder aus dem System destillativ entfernt werden können, d. h. bei einem Druck von 0,25 bis 70 bar, bevorzugt 0,35 bis 50 bar, besonders bevorzugt 0,5 bis 30 bar.

Die Behandlung gemäß Stufe (e1) kann diskontinuierlich oder kontinuierlich erfolgen, wobei eine kontinuierliche Behandlung bevorzugt ist. Die Verweilzeiten liegen dabei beispielsweise zwischen 0,1 und 50 Stunden, bevorzugt zwischen 0,2 und 24 Stunden, beispielsweise zwischen 0,5 und 15 Stunden, insbesondere zwischen 1 Stunde und 19 Stunden, besonders bevorzugt zwischen 1,5 und 10 Stunden.

Daher betrifft die vorliegende Erfindung gemäß einer weiteren Ausführungsform auch ein wie zuvor beschriebenes Verfahren zur Herstellung einer cyclischen Verbindung mit Z Cyclen und 7 bis 16 C-Atomen und einer Ketogruppe, wobei die Behandlung gemäß Schritt (e1) für eine Zeit von 0,1 bis 50 Stunden durchgeführt wird.

Die erfindungsgemäß eingesetzten Säuren sind Brønstedt oder Lewis-Säuren, wobei auch Gemische aus zwei oder mehr Säuren eingesetzt werden können. Die eingesetzten Säuren können homogen gelöst oder heterogen sein. Heterogene Säuren können erfindungsgemäß suspendiert oder fest angeordnet sein.

Daher betrifft die vorliegende Erfindung gemäß einer weiteren Ausführungsform auch ein wie zuvor beschriebenes Verfahren, wobei die Säure homogen oder heterogen vorliegt.

Die erfindungsgemäß eingesetzten homogen löslichen Säuren sind beispielsweise Mineralsäuren oder organische Säuren. Beispiele sind Schwefelsäure, Sulfonsäuren, Salpetersäure, Salzsäure, Phosphorsäure, Phosphorige Säure, Perchlorsäure, Heteropolysäuren wie sie beispielsweise in EP 0 158 229 B1 beschrieben sind, C1 bis C30 Carbonsäuren wie Ameisensäure, Essigsäure, Propionsäure, Benzoesäure oder ähnlich.

Bevorzugte homogene Säuren sind Phosphorsäure, Phosphorige Säure, Schwefelsäure, Sulfonsäuren und Heteropolysäuren wie z. B. Wolframatophosphorsäure. Besonders bevorzugt sind Phosphorsäure und Wolframatophosphorsäure.

Der Gehalt an homogen löslicher Säure beträgt in der Regel zwischen 0,01 und 10 Gew.-% bezogen auf das cyclische Keton. Bevorzugt wird eine homogen lösliche Säure in einer Menge von 0,05 bis 5 Gew.-%, besonders bevorzugt 0,1 bis 1 Gew.-%, eingesetzt.

Gemäß einer bevorzugten Ausführungsform der vorliegenden Erfindung wird eine homögen lösliche Säure in einer Menge von 0,1 bis 1 Gew.-% eingesetzt.

Bevorzugt wird die Säure nach destillativer Abtrennung der mindestens einen cyclischen Verbindung mit Z Cyclen und 7 bis 16 C-Atomen und einer Ketogruppe cyclischen Ketons, zumindest zum Teil in die Behandlungsstufe rückgeführt.

Erfindungsgemäß geeignete heterogene Säuren sind beispielsweise metalloxidische Feststoffe, die erfindungsgemäß zur Erhöhung ihrer Säurestärke beispielsweise mit Mineralsäuren wie Phosphorsäure oder Schwefelsäure behandelt worden sein können. Bevorzugt werden Oxide oder Mischoxide von B, Al, Si, Sn, Ti, Cr, Zr, Fe und Zn eingesetzt, die noch weitere Bestandteile enthalten können. Beispielsweise geeignet sind Zirkonoxid, Titanoxid, Aluminiumoxid, Siliziumoxid und Kombinationen daraus wie Alumosilikate wie z. B. Zeolithe. Einsetzbar sind beispielsweise Schichtsilikate oder natürliche Tonerden. Ebenfalls einsetzbar sind heterogene Säuren auf organischer Basis, wie z. B. saure lonentauscher.

Wird Schritt (e1) des erfindungsgemäßen Verfahrens mit heterogenen Säuren diskontinuierlich durchgeführt, so wird in der Regel 0,1 bis 50 Gew.-% Säure bezogen auf die mindestens eine cyclische Verbindung mit Z Cyclen und 7 bis 16 C-Atomen und einer Ketogruppe eingesetzt. Bevorzugt wird eine heterogene Säure in einer Menge von 0,5 bis 20 Gew.-%, besonders bevorzugt von 1 bis 10 Gew.-% eingesetzt.

Wird Schritt (e1) des erfindungsgemäßen Verfahrens kontinuierlich mit einer heterogenen Säure durchgeführt, so wird vorzugsweise eine Katalysatorbelastung, d. h. die Belastung der heterogenen Säure, von 0,01 bis 10 kg mindestens eine cyclische Verbindung mit 7 bis 16 C-Atomen und einer Ketogruppe / Liter Katalysator / h eingestellt. Insbesondere wird eine Katalysatorbelastung von 0,05 bis 2 kg cyclische Verbindung / Liter Katalysator / h, besonders bevorzugt 0,1 bis 1 kg cyclische Verbindung / Liter Katalysator / h eingestellt.

Die erfindungsgemäß eingesetzten Katalysatoren enthalten mindestens ein Übergangsmetall, wobei auch Katalysatoren enthaltend zwei oder mehrere Übergangsmetalle oder Gemische aus zwei oder mehreren Katalysatoren enthaltend mindestens ein Übergangsmetall eingesetzt werden können. Die eingesetzten Katalysatoren können homogen gelöst oder heterogen sein. Heterogene Katalysatoren können erfindungsgemäß suspendiert oder fest angeordnet sein. Daher betrifft die vorliegende Erfindung gemäß einer weiteren Ausführungsform auch ein wie zuvor beschriebenes Verfahren, wobei der Katalysator, der mindestens ein Übergangsmetall enthält, homogen oder heterogen vorliegt.

Als Katalysator enthaltend mindestens ein Übergangsmetall können im Rahmen der vorliegenden Erfindung alle üblichen Katalysatoren eingesetzt werden. Als Übergangsmetall kommen dabei prinzipiell alle dem Fachmann bekannten Übergangsmetalle in Frage.

Die erfindungsgemäß eingesetzten homogen löslichen Katalysatoren sind beispielsweise in Houben-Weyl, Methoden der Organischen Chemie, Band IV/1 c, Seiten 45 bis 67, Thieme Verlag Stuttgart, 1980, beschrieben.

Bevorzugte homogene Katalysatoren enthalten Ru, Rh und/oder Pd als Übergangsmetall. Besonders bevorzugt ist Ru.

Der Gehalt an homogen löslichem Katalysator beträgt in der Regel zwischen 0,001 und 1 Gew.-%, bezogen auf die cyclische Verbindung. Bevorzugt wird ein homogen löslicher Katalysator in einer Menge von 0,005 bis 0,5 Gew.-%, besonders bevorzugt 0,01 bis 0,1 Gew.-% eingesetzt.

Gemäß einer bevorzugten Ausführungsform der vorliegenden Erfindung wird ein homogen löslicher Katalysator in einer Menge von 0,01 bis 0,1 Gew.-% eingesetzt.

Bevorzugt wird der Katalysator nach destillativer Abtrennung der mindestens einen cyclischen Verbindung mit Z Cyclen und 7 bis 16 C-Atomen und einer Ketogruppe zumindest zum Teil in die Behandlungsstufe (e1) rückgeführt.

Erfindungsgemäß geeignete heterogene Katalysatoren sind beispielsweise in Houben-Weyl, Methoden der Organischen Chemie, Band IV/1c, S. 16 bis 26, Thieme Verlag Stuttgart, 1980, beschrieben. Sie enthalten mindestens ein Übergangsmetall. Bevorzugte Übergangsmetalle sind Ni, Cu, Pd, Ru, Ir, Pt, Co und/oder Rh. Besonders bevorzugt sind Pd, Ru, Pt, ganz besonders bevorzugt sind Ru und Pd.

Die heterogenen Katalysatoren können in suspendierter Form oder bevorzugt fest angeordnet eingesetzt werden. Die Katalysatoren enthaltend mindestens ein Übergangsmetall können das Übergangsmetall als Element oder in Form einer chemischen Verbindung, beispielsweise als Oxid enthalten. Mischungen von verschiedenen Übergangsmetallen können die Elemente oder deren Verbindungen als Mischungen oder als Legierungen enthalten. Dabei können auch nicht übergangsmetallhaltige Elemente oder deren Verbindungen als Katalysatorkomponente verwendet werden, beispielsweise in den so genannten Raney-Katalysatoren, wobei beispielsweise Al oder Aluminiumoxid, bevorzugt zusammen mit Ni, Cu oder Ru eingesetzt werden.

Erfindungsgemäß geeignet sind beispielsweise auch Ru auf Aluminium-, Silizium-, Titan-, Zirkon-, Barium-, Calziumoxid, Ru auf Aktivkohle, Pd auf Aluminium-, Silizium-, Titan-, Zirkon-, Barium-, Calziumoxid, Pd auf Aktivkohle, Pt auf Aluminium-, Silizium-, Titan-, Zirkon-, Barium-, Calziumoxid oder Pt auf Aktivkohle. Als Trägermaterialien können auch Mischungen oder Verbindungen verschiedener Materialen verwendet werden, beispielsweise Tonerden oder Zeolithe.

Erfindungsgemäß geeignet sind auch beispielsweise die in der Hydrierung eingesetzten Katalysatoren.

Die Katalysatoren enthaltend mindestens ein Übergangsmetall können erfindungsgemäß auch auf einem Träger aufgebracht sein. Diese Träger sind beispielsweise metalloxidische, basische, neutrale oder saure Feststoffe, die erfindungsgemäß zur Erhöhung der Säurestärke beispielsweise mit Mineralsäuren wie Phosphorsäure oder Schwefelsäure behandelt worden sein können. Bevorzugt werden Oxide oder Mischoxide von B, Al, Si, Sn, Ti, Cr, Zr, Fe und Zn eingesetzt, die noch weitere Bestandteile enthalten können. Beispielsweise geeignet sind Zirkonoxid, Titanoxid, Aluminiumoxid, Siliziumoxid und Kombinationen daraus wie Alumosilikate, wie beispielsweise Zeolithe. Einsetzbar sind beispielsweise Schichtsilikate oder natürliche Tonerden.

Wird das erfindungsgemäße Verfahren mit heterogenen Katalysatoren diskontinuierlich durchgeführt, so wird in der Regel 0,1 bis 50 Gew.-% Katalysator bezogen auf die mindestens eine cyclische Verbindung mit Z Cyclen und 7 bis 16 C-Atomen mit einer Ketogruppe eingesetzt. Bevorzugt wird ein heterogener Katalysator in einer Menge von 0,5 bis 20 Gew.-%, besonders bevorzugt von 1 bis 10 Gew.-% eingesetzt.

Wird das Verfahren gemäß Stufe (e1) kontinuierlich mit einem heterogenen Katalysator durchgeführt, so wird vorzugsweise eine Katalysatorbelastung, d. h. die Belastung des heterogenen Katalysators, von 0,01 bis 10 kg mindestens eine cyclische Verbindung mit 7 bis 16 C-Atomen und einer Ketogruppe / Liter Katalysator / h eingestellt. Insbesondere wird eine Katalysatorbelastung von 0,05 bis 2 kg mindestens eine cyclische Verbindung mit 7 bis 16 C-Atomen und einer Ketogruppe / Liter Katalysator / h, besonders bevorzugt 0,1 bis 1 kg mindestens eine cyclische Verbindung mit Z Cyclen und 7 bis 16 C-Atomen und einer Ketogruppe / Liter Katalysator / h eingestellt.

Erfindungsgemäß ist es möglich, dass die Säure oder der Katalysator, der mindestens ein Übergangsmetall enthält, im Schritt (e2) abgetrennt wird. Ebenso ist es jedoch im Rahmen der vorliegenden Erfindung möglich, dass die Säure oder der Katalysator nach Schritt (e1) und vor Schritt (e2) abgetrennt wird. Mögliche Verfahren zur Abtrennung sind beispielsweise Destillation, Extraktion, Fällung oder Kristallisation.

Gemäß Stufe (e2) wird die derart behandelte Zusammensetzung durch Destillation, Extraktion und/oder Kristallisation weiter aufgereinigt. Dabei können die Destillation, Extraktion und/oder Kristallisation nach allen dem Fachmann bekannten üblichen Verfahren durchgeführt werden.

Geeignete Lösungsmittel für die Kristallisation gemäß Stufe (e2) sind beispielsweise Alkohole, Ether, Kohlenwasserstoffe, aromatische Kohlenwasserstoffe, Ketone, bevorzugt Toluol, Xylol, Methanol, Ethanol, Propanol, Butanol, Aceton, Diethylketon oder Methyl-tert.-butylether. Erfindungsgemäß ist es ebenso möglich, dass kein Lösungsmittel verwendet wird, sondern eine Schmelzkristallisation durchgeführt wird.

Die destillative Reinigung kann in einer oder mehreren Kolonnen erfolgen. Dabei wird bevorzugt bei Drücken zwischen 1 und 2000 mbar gearbeitet. Besonders bei cyclischen Verbindungen mit einer Ketogruppe und mehr als 8 Kohlenstoffatomen sind Drücke zwischen 5 und 500 mbar bevorzugt, besonders bevorzugt sind 10 bis 200 mbar. Die Temperaturen (Sumpftemperatur) liegen bei 100 bis 300 °C. Bevorzugt liegt die Temperatur bei der destillativen Reinigung bei 130 bis 250 °C, besonders bevorzugt bei 150 bis 220 °C.

Gemäß einer bevorzugten Ausführungsform der Erfindung wird die destillative Reinigung bei einem Druck von 1 bis 2000 mbar, bevorzugt 5 bis 500 mbar, besonders bevorzugt 10 bis 200 mbar, und einer Sumpftemperatur von 100 bis 300 °C, bevorzugt 130 bis 250 °C, besonders bevorzugt 150 bis 220 °C durchgeführt.

Sofern bei der destillativen Reinigung eine Kolonne verwendet wird, wird das Wertprodukt bevorzugt über einen Seitenabzug gewonnen. Dabei ist es erfindungsgemäß möglich, das gewünschte Produkt flüssig oder gasförmig zu erhalten. Über Sumpf werden vorzugsweise Schwersieder, über Kopf vorzugsweise Leichtsieder abgetrennt. Werden zwei Kolonnen verwendet, so geht das Wertprodukt bevorzugt zusammen mit Schwersiedern bevorzugt über Sumpf in die zweite Kolonne, aus der es dann über Kopf oder wiederum als Seitenabzug gewonnen werden kann. Erfindungsgemäß können auch Trennwandkolonnen zum Einsatz kommen.

Dabei ist es erfindungsgemäß auch möglich, dass zwischen den einzelnen Schritten des Verfahrens weitere Behandlungen erfolgen. Insbesondere ist es erfindungsgemäß möglich, nach der Stufe (e1) die Säure oder den Katalysator, der mindestens ein Übergangsmetall enthält, abzutrennen.

Vor der Destillation, Extraktion bzw. Kristallisation gemäß Stufe (e2) kann es vorteilhaft sein, die Säure oder den Katalysator, der mindestens ein Übergangsmetall enthält, aus der in Stufe (e) behandelten Zusammensetzung (C) oder (C') zu entfernen, insbesondere dann, wenn der Katalysator homogen gelöst vorliegt. Dies kann im Fall von heterogenen Säuren oder Katalysatoren beispielsweise durch Filtration geschehen, im Fall von homogenen Säuren oder Katalysatoren bieten sich beispielsweise Extraktion, beispielsweise mit Wasser, oder eine Destillation an, wobei die Säure, je nach Siedepunkt, über Kopf oder über Sumpf abgetrennt wird, und der Katalysator vorzugsweise über Sumpf abgetrennt wird.

Vorteilhafterweise kann die Säure oder der Katalysator nach der Abtrennung erneut in die Stufe (e1) eingesetzt werden. Erfindungsgemäß ist es auch möglich, dass die Säure oder der Katalysator nach der Abtrennung einer Zwischenbehandlung, beispielsweise einer Reinigung, unterzogen wird, bevor er erneut in die Stufe (e1) eingesetzt wird.

Durch die erfindungsgemäß bevorzugte Kombination einer destillativen Aufreinigung, einer Behandlung mit einer Base, bei der insbesondere Aldehyde angegriffen werden, und der Behandlung mit einer Säure oder einem Übergangsmetall enthaltenen Katalysator, bei der überwiegend Epoxide und Alkohole angegriffen werden, werden besonders reine Produkte erhalten. Dabei ist das erfindungsgemäße Verfahren apparativ einfach und kostengünstig.

In einer ganz besonders bevorzugten Ausführungsform umfasst das erfindungsgemäße Verfahren die folgenden Stufen:
(a1) Oxidation einer Zusammensetzung (A), mindestens enthaltend ein cyclisches Olefin mit Z Cyclen und 7 bis 16 C-Atomen und mindestens zwei C-C-Doppelbindungen, mittels Distickstoffmonoxid unter Erhalt einer Zusammensetzung (A1), mindestens enthaltend
   - die mindestens eine cyclische Verbindung mit Z Cyclen und 7 bis 16 C-Atomen mit einer Ketogruppe,
   - mindestens eine cyclische Verbindung mit Z Cyclen und 7 bis 16 C-Atomen mit mindestens zwei Ketogruppen,
   - das mindestens eine cyclische Olefin mit Z Cyclen und 7 bis 16 C-Atomen mit mindestens zwei C-C-Doppelbindungen,
   - mindestens eine Verbindung mit Z-1 Cyclen und 7 bis 16 C-Atomen mit mindestens einer Aldehydgruppe und
   - Distickfoffmonoxid und Stickstoff,
(a1b) Entspannen der Zusammensetzung (A1) aus Schritt (a1), um Distickstoffmonoxid und Stickstoff zu entfernen, um eine Zusammensetzung (A1) zu erhalten, die im Wesentlichen frei von Distickstoffmonoxid und Stickstoff ist,
(a2) Abtrennen des mindestens einen cyclischen Olefins mit Z Cyclen und 7 bis 16 C-Atomen mit mindestens zwei C-C-Doppelbindungen aus der Zusammensetzung (A1) aus Stufe (a1b), um eine Zusammensetzung (A2) zu erhalten, mindestens enthaltend
   - die mindestens eine cyclische Verbindung mit Z Cyclen und 7 bis 16 C-Atomen mit einer Ketogruppe,
   - mindestens eine cyclische Verbindung mit Z Cyclen und 7 bis 16 C-Atomen mit mindestens zwei Ketogruppen und
   - mindestens eine Verbindung mit Z-1 Cyclen und 7 bis 16 C-Atomen mit mindestens einer Aldehydgruppe, und
(b) destillative Behandlung der Zusammensetzung (A2) aus Schritt (a2), um eine Zusammensetzung (B) zu erhalten, enthaltend
   - die mindestens eine cyclische Verbindung mit Z Cyclen und 7 bis 16 C-Atomen mit einer Ketogruppe,
   - weniger als 0,5 Gew.-% der mindestens einen cyclischen Verbindung mit Z Cyclen und 7 bis 16 C-Atomen mit mindestens zwei Ketogruppen und
   - weniger als 1,0 Gew.-% der mindestens einen Verbindung mit Z-1 Cyclen und 7 bis 16 C-Atomen mit mindestens einer Aldehydgruppe,
(c) Hydrierung der aus Schritt (b) erhaltenen Zusammensetzung (B) in Gegenwart mindestens eines Katalysators, um eine Zusammensetzung (C) zu erhalten und
(e) Reinigen der Zusammensetzung (C) aus Schritt (c), mindestens umfassend die Schritte
   (e1) thermisches Behandeln der Zusammensetzung (B) mit mindestens einer Säure, oder mindestens einem Katalysator, der mindestens ein Übergangsmetall enthält,
   (e2) weiteres Aufreinigen durch eine Destillation,
   um eine gesättigte cyclische Verbindung mit Z Cyclen und 7 bis 16 C-Atomen und einer Ketogruppe, wie oben definiert, insbesondere Cyclododecanon, zu erhalten.

Im Folgenden wird die vorliegende Erfindung anhand von Beispielen näher erläutert.

### Beispiele:

### Beispiel 1: Oxidation von 1,5,9-Cyclododecatrien (CDT) mit N₂O und Abtrennung von nicht umgesetztem 1,5,9-Cyclododecatrien (Stufen (a1) und (a2))

Aus entsprechenden Vorlagebehältern und mittels geeigneter Dosierpumpen werden 2000 g/h 1,5,9-Cyclododecatrien und 68 g/h flüssiges N₂O über einen statischen Mischer in einen Rohrreaktor (Doppelmantelrohr, gewickelt, Øᵢₙₙₑₙ = 6 mm, Länge 36 m) gepumpt. Das Rohr wird mittels Wärmeträgeröl, das im Doppelmantel im Gleichstrom zum Produkt fließt, auf 280 °C thermostatisiert, wobei die Ölausgangstemperatur weniger als 2 °C über der Öleingangstemperatur liegt. Der Reaktionsdruck wird mittels eines Druckregelventils am Reaktorausgang auf 100 bar geregelt. Der Umsatz an 1,5,9-Cyclododecatrien am Reaktorausgang beträgt 11,3%. Nach Passieren der Reaktionszone wird das Reaktionsgemisch in zwei unisolierten Flash-Behältern zunächst auf 3 bar und anschließend auf 60 mbar entspannt, um das gebildete N₂ und nicht umgesetztes N₂O abzuführen. Dabei kühlt das Produkt auf unter 100 °C ab.

Das flüssige Produkt wird dann in einer Packungskolonne mit mindestens 7 theoretischen Trennstufen bei 60 mbar Kopfdruck destilliert (T_{Sumpf} = 170 °C, T_{Kopf} = 130 °C). Als Kopfprodukt erhält man nicht umgesetztes 1,5,9-Cyclododecatrien mit einer Reinheit > 99%, das wieder in die Reaktion zurückgeführt wird. Der Sumpfaustrag ist eine nur leicht gelbliche Flüssigkeit und hat eine Zusammensetzung gemäß Tabelle 1:

**Tabelle 1**

| **Verbindung** | **Menge [Gew.-%]** |
|---|---|
| Cyclododeca-4,8-dienone | 92,0 |
| 4,8,11-Dodecatrienale | 2,3 |
| Cyclododecendione | 2,2 |
| Cyclo-undeca-3,7-diencarbaldehyd | 1,0 |
| 1,5,9-Cyclododecatrien | 0,4 |
| trans-1,2-Epoxy-cis,trans-5,9-cyclododecadien | 0,01 |
| Dimere | 2,0 |
| andere, nicht identifiziert | Rest zu 100 |

Das Produkt wird gesammelt und in Beispiel 2 eingesetzt.

### Beispiel 2: Stufe (b)

Für die Destillation des Produktgemisches aus Beispiel 1 wird eine kontinuierliche Labortrennwandkolonne mit 64 mm Innendurchmesser und einer Länge von 2,6 m (Gesamtlänge der Packung) verwendet. In Vorversuchen mit Testgemischen wird ermittelt, dass die Kolonne 35 theoretische Trennstufen hat. Die Kolonne ist in drei Bereiche unterteilt. Der untere Bereich (Stufen 1 bis 9) hat eine Länge von 0,65 m. Der mittlere Bereich (Stufen 9 bis 27) ist 1,3 m lang und durch eine mittig angeordnete Trennwand in eine Eingangs- und einer Ausgangsseite geteilt. Auf der Eingangsseite ist der Zulauf auf der Höhe von Stufe 19 angebracht. Auf der Ausgangsseite wird auf der Höhe der Stufe 12 das Seitenstromprodukt gasförmig abgenommen. Der obere Bereich (Stufen 27 bis 35) hat eine Länge von 0,65 m. Die gesamte Kolonne ist mit einer Packung bestückt (Montz A3 750). Die Destillation wird bei einem Kopfdruck von ca. 44 mbar durchgeführt, der Druckverlust über die Packung beträgt 3,6 mbar. Um die Verweilzeit und damit die thermische Belastung im Sumpf zu minimieren, wird als Sumpfverdampfer ein Sambay-Verdampfer (*wiped film evaporator*) eingesetzt. Die Kopftemperatur beträgt 137 °C und die Sumpftemperatur 185 °C. Mit einer Dosierpumpe werden 501 g/h des zu destillierenden Gemisches dosiert, wobei das Gemisch vorher auf 180 °C erhitzt worden ist. Über den Seitenstrom werden 481 g/h Produkt erhalten, das die Zusammensetzung gemäß Tabelle 2 aufweist (Gew.-%).

**Tabelle 2**

| **Verbindung** | **Menge [Gew.-%]** |
|---|---|
| Cyclododeca-4,8-dienone | 98,5 |
| 4,8,11-Dodecatrienale | 0,1 |
| Cyclododecendione | 0,2 |
| Cyclo-undeca-3,7-diencarbaldehyd | 1,1 |
| 1,5,9-Cyclododecatrien | 0,4 |
| trans-1,2-Epoxy-cis,trans-5,9-cyclododecadien | 0,01 |

Es handelt sich hierbei um eine farblose Flüssigkeit mit einem Schmelzpunkt von +1°C.

Im Sumpf werden 6 g/h Sumpfprodukt als dunkelgelbe bis braune Flüssigkeit mit einer Zusammensetzung gemäß Tabelle 3 erhalten (Gew.-%).

**Tabelle 3**

| **Verbindung** | **Menge [Gew.-%]** |
|---|---|
| Cyclododeca-4,8-dienone | 6,7 |
| Cyclododecendione | 47,0 |
| Hochsieder | 46,0 |

Im Kopf werden 14 g/h Kopfprodukt als farblose Flüssigkeit mit der Zusammensetzung gemäß Tabelle 4 erhalten (Gew.-%).

**Tabelle 4**

| **Verbindung** | **Menge [Gew.-%]** |
|---|---|
| 4,8,11-Dodecatrienale | 76,0 |
| Cyclododeca-4,8-dienone | 9,9 |
| 1,5,9-Cyclododecatrien | 13,6 |

Die angegebenen Flüsse sind Mittelwerte aus einer kontinuierlichen Destillation, bei der insgesamt 80 kg Zulauf verarbeitet wird.

### Beispiel 3 (Vergleichsbeispiel)

### Behandlung von Cyclododeca-4,8-dienonen mit Base ohne Destillation

550 g vom Sumpfaustrag aus Beispiel 1 werden in einem Rührkolben vorgelegt und unter Schutzgas (N₂) auf 160 °C aufgeheizt. Anschließend werden mit einer Spritze 2,75 g einer 25 %-igen wässrigen NaOH-Lösung zugegeben. Die Reaktionsmischung bleibt dabei klar und homogen. In regelmäßigen Abständen werden Proben entnommen und mittels GC analysiert. Nach 95 min enthält die Lösung nur noch < 30 Gew.-ppm Dodeca-4,8,11-trienal und 820 Gew.-ppm Cycloundeca-3,7-diencarbaldehyd. Der Gehalt an Cyclododeca-4,8-dienon sinkt auf 88,5 Gew.-%. Das bedeutet, dass ca. 4% des Wertproduktes bei der Basenbehandlung zerstört werden.

Auch die Cyclododecendione werden durch die Basenbehandlung abgebaut. Die Cyclododecendione werden abgebaut und an deren Stelle werden bizyklische Dienone gebildet. Nach der Hydrierung würden daraus bizyklische C₁₂-Ketone entstehen, von denen bekannt ist, dass sie sich aufgrund des sehr ähnlichen Siedepunktes destillativ schwer von Cyclododecanon abtrennen lassen. Als Beispiel dient das Decahydroheptalenon mit einem Siedepunkt von 145 °C bei 15 Torr (G. Büchi, O. Jeger, Helv. Chim. Acta (1949) 32, 538). Bei diesem Druck hat Cyclododecanon einen Siedepunkt von 140 °C und liegt demnach sehr nah an dem Siedepunkt von Cyclododecanon.

### Beispiel 4: Behandlung von Cyclododeca-4,8-dienonen mit Base

550 g vom Seitenabzugsprodukt aus Beispiel 2 werden in einem Rührkolben vorgelegt und unter Schutzgas (N₂) auf 160 °C aufgeheizt. Anschließend werden mit einer Spritze 2,75 g einer 25 %-igen wässrigen NaOH-Lösung zugegeben. Die Reaktionsmischung bleibt dabei klar und homogen. In regelmäßigen Abständen werden Proben entnommen und mittels GC analysiert. Nach 95 min enthält die Lösung keine Dodeca-4,8,11-trienale, und nur 950 Gew.-ppm Cycloundeca-3,7-diencarbaldehyd. Der Gehalt an Cyclododeca-4,8-dienon sinkt aber lediglich auf 97,4 Gew.-%. Das bedeutet, dass nur ca. 1% des Wertproduktes bei der Basenbehandlung zerstört werden (statt 4% in Beispiel 3, Vergleichbeispiel).

### Beispiel 5 (Vergleichsbeispiel):

### Hydrierung von Cyclododeca-4,8-dienon ohne vorherige Destillation

Für die Hydrierung wird einer Reaktorkaskade, bestehend aus einem Rohreaktor (1,6 m Länge, 20 mm Innendurchmesser, 350 ml Katalysatorbettvolumen) mit Flüssigumlauf und einem Nachreaktor (15 m Gesamtlänge, verteilt auf 12 Segmente, 4 mm Innendurchmesser, 150 ml Katalysatorbettvolumen) eingesetzt, wobei beide Reaktoren mit einem Doppelmantel, über dem einen Wärmeträgeröl zwecks Thermostatisierung gekreist wird, versehen sind. Die Reaktoren sind mit einem Pd (0,2 Gew.-%) auf Aluminiumoxid-Katalysator (3 mm Stränge, Pd als wässrige Palladiumnitrat-Lösung auf einem kommerziellen Aluminiumoxidträger aufgetränkt, dann getrocknet und bei 300 °C an der Luft kalziniert) gefüllt. In diese Anlage wird das Sumpfprodukt aus Beispiel 1 bei 130 °C und 30 bar Wasserstoffdruck hydriert, wobei 3 Mole Wasserstoff/mol Feed, der als 100% Cyclododeca-4,8-1-on gerechnet wird, eingesetzt werden. Die Reaktoren werden in Rieselfahrweise betrieben. Das Verhältnis Flüssigumlauf zu Feed im ersten Reaktor beträgt 10 zu 1 und die Gesamtkatalysatorbelastung 0,5 kg Feed/Liter Katalysator/h. Sowohl flüssiger als auch gasförmiger Feed werden zuerst in den Hauptreaktor eingebracht. Anschließend werden sowohl der flüssige als auch der gasförmige Austrag aus dem Hauptreaktor direkt zum Nachreaktor geleitet. Die Hydrierung wird 300 h lang betrieben. Der Hydrieraustrag weist die Zusammensetzung gemäß Tabelle 5 auf (Gew.-%).

**Tabelle 5**

| **Verbindung** | **Menge [Gew.-%]** |
|---|---|
| Cyclododecanon | 91,5 |
| Cyclododecenone | 0,3 |
| Cyclododecanol | 0,2 |
| Dodecanal | 1,9 |
| 1-Dodecanol | 0,2 |
| Cycloundecancarbaldehyd | 0,7 |
| Hydroxymethylcycloundecan | 0,2 |
| andere Verbindungen, nicht ident. | jeweils < 0,1, Summe Rest zu 100 |

Dieses Produkt wird dann über ein saures Titandioxid (1,5 mm Stränge, reinem Titandioxid zu mehr als 99% in der Anatas-Form wie in der WO2007/104650 beschrieben, 315 ml Katalysatorbettvolumen, Rohrreaktor mit einer Länge von 1,5 m, Heizung durch außenliegenden Ölheizmantel) bei 200 °C und Normaldruck geleitet (Katalysatorbelastung ca. 0,5 kg Feed/Liter Katalysator/h). Dieser Austrag wird gesammelt und 3 kg davon in einer Destillationsblase mit aufgesetzter Kolonne (1 m Füllkörperkolonne) fraktioniert destilliert. Die Fraktion (0,5 kg) mit der höchsten Cyclododecanon-Reinheit (99,5 Gew.-%) (ca. 50 mbar Destillationsdruck, ca. 165 °C Siedepunkt) enthält noch die Nebenkomponenten gemäß Tabelle 6 (Gew.-%):

**Tabelle 6**

| **Verbindung** | **Menge [Gew.-%]** |
|---|---|
| Cyclododecenone | 0,3 |
| Cycloundecancarbaldehyd | 0,02 |
| Hydroxymethylcycloundecan | 0,01 |
| andere Verbindungen, nicht ident. | jeweils < 0,05, Summe Rest zu 100 |

### Beispiel 6 (Referenz)

### Hydrierung von Cyclododeca-4,8-dienon mit vorheriger Destillation

Beispiel 5 wird wiederholt, mit dem Unterschied, dass als Edukt das Seitenabzugsprodukt aus Beispiel 2 eingesetzt wird. Die Hydrierausträge haben die Zusammensetzung gemäß Tabelle 7 (Gew.-%).

**Tabelle 7**

| **Verbindung** | **Menge [Gew.-%]** |
|---|---|
| Cyclododecanon | 98,4 |
| Cyclododecanol | 0,1 |
| Dodecanal | 0,05 |
| 1-Dodecanol | 0,04 |
| Cycloundecancarbaldehyd | 0,8 |
| Hydroxymethylcycloundecan | 0,2 |
| andere Verbindungen, nicht ident. | jeweils < 0,05, Summe Rest zu 100 |

Dieses Produkt wird dann über saures Titandioxid bei 200°C und Normaldruck geleitet (Katalysatorbelastung ca. 1 kg Feed/Liter Katalysator/h). Dieser Austrag wird gesammelt und 3 kg davon in einer Destillationsblase mit aufgesetzter Kolonne fraktioniert destilliert. Die Fraktion (2,5 kg) mit der höchsten Cyclododecanon-Reinheit (99,9 Gew.-%) enthält noch die Nebenkomponenten gemäß Tabelle 8 (Gew.-%):

**Tabelle 8**

| **Verbindung** | **Menge [Gew.-%]** |
|---|---|
| Cycloundecancarbaldehyd | 0,01 |
| Hydroxymethylcycloundecan | 0,01 |
| andere Verbindungen, nicht ident. | jeweils < 0,005, Summe Rest zu 100 |

### Beispiel 7

### Oxidation von 1,5-Cyclooctadien mit N₂O

1,5-Cyclooctadien (kommerzielles Produkt, Aldrich mind. 99% redestilliert, 700 mL) wurde in einen 1,2L-Autoklaven eingefüllt, der Autoklav verschlossen und mit N₂ inertisiert. Der Autoklav wurde dreimal mit 50 bar N₂ gespült und anschließend wird mit N₂O ohne Rührerbewegung auf 50 bar Kaltdruck aufgepresst. Der Rührer wurde eingeschaltet, auf 400 U/min eingestellt und anschließend auf Solltemperatur (200 °C) für 24h erwärmt. Nach 24 h wurde der Autoklav auf Raumtemperatur abgekühlt und auf Umgebungsdruck entspannt. Anschließend wurde der Vorgang des Inertisierens, der N₂O-Zugabe, des Erwärmens auf 200 °C und der Entspannung noch zweimal wiederholt.

### Austrag:

**Tabelle 9**

| **Verbindung** | **Menge [FI.-%]** |
|---|---|
| 1,5-Cyclooctadien (Startmaterial) | 17,8 |
| Cyclooct-4-en-1-on | 34,1 |
| 1,5-Cyclooctadion | 21,5 |
| 3a-Hydroxy-hexahydro-pentalen-1-on | 18,4 |
| (Z)-Octa-4,7-dienal | 0,19 |
| Cyclohept-4-encarbaldehyd | 0,16 |
| andere Verbindungen (u.a. Dimere), nicht ident. | jeweils < 0,2, Summe Rest zu 100 |

### Beispiel 8

### Oxidation von Cyclotetradeca-1,8-dien

Cyclotetradeca-1,8-dien (Isomerengemisch, ca. 91%, 2 g) und Cyclohexan (98 g) wurden in einen 300mL-Autoklaven eingefüllt, der Autoklav verschlossen und mit N₂ inertisiert. Der Autoklav wurde dreimal mit 50 bar N₂ gespült und anschließend wird mit N₂O ohne Rührerbewegung auf 30 bar Kaltdruck aufgepresst. Der Rührer wurde eingeschaltet, auf 400 U/min eingestellt und anschließend auf Solltemperatur (280 °C) erwärmt. Nach 24 h wurde der Autoklav auf Raumtemperatur abgekühlt, auf Umgebungsdruck entspannt und das Produkt per GC analysiert.

### Austrag:

**Tabelle 10**

| **Verbindung** | **Menge [FI.-% abzüglich Lsgmtl.]** |
|---|---|
| Cyclotetradeca-1,8-dien (Startmaterial, 2 Iso.) | 11,2 |
| Cyclotetradec-7-en-1-on (2 Iso.) | 12,1 |
| Cyclotetradeca-1,7-dion | 29,1 |
| Cyclotetradeca-1,8-dion | 27,6 |
| Tetradeca-1,8-dienal | 1,7 |
| Cyclotridec-7-encarbaldehyd | 1,2 |
| andere Verbindungen, nicht ident. | jeweils < 0,5, Summe Rest zu 100 |

### Beispiel 9

### Oxidation von Cyclohexadeca-1,9-dien

Cyclohexadeca-1,9-dien (Isomerengemisch, ca. 89%, 2,1 g) und Cyclohexan (98,5 g) wurden in einen 300mL-Autoklaven eingefüllt, der Autoklav verschlossen und mit N₂ inertisiert. Der Autoklav wurde dreimal mit 50 bar N₂ gespült und anschließend wird mit N₂O ohne Rührerbewegung auf 30 bar Kaltdruck aufgepresst. Der Rührer wurde eingeschaltet, auf 400 U/min eingestellt und anschließend auf Solltemperatur (240 °C) erwärmt. Nach 24 h wurde der Autoklav auf Raumtemperatur abgekühlt, auf Umgebungsdruck entspannt und das Produkt per GC analysiert.

### Austrag:

**Tabelle 11**

| **Verbindung** | **Menge [FI.-% ohne Lsgmtl.]** |
|---|---|
| Cyclohexadeca-1,9-dien (Startmaterial, 3 Iso.) | 21,6 |
| Cyclohexadec-8-en-1-on (2 Iso.) | 33,7 |
| Cyclohexadecandion (2 Iso.) | 13,6 |
| Hexadeca-8,15-dienal | 1,0 |
| andere Verbindungen, nicht ident. | jeweils < 0,2, Summe Rest zu 100 |

## Patentansprüche

1. Verfahren zur Herstellung mindestens einer cyclischen Verbindung mit Z Cyclen und 7 bis 16 C-Atomen mit einer Ketogruppe, mindestens umfassend die Stufen:
(a1) Oxidation einer Zusammensetzung (A), mindestens enthaltend ein cyclisches Olefin mit Z Cyclen und 7 bis 16 C-Atomen und mindestens zwei C-C-Doppelbindungen, mittels Distickstoffmonoxid unter Erhalt einer Zusammensetzung (A1), mindestens enthaltend
- die mindestens eine cyclische Verbindung mit Z Cyclen und 7 bis 16 C-Atomen mit einer Ketogruppe,
- mindestens eine cyclische Verbindung mit Z Cyclen und 7 bis 16 C-Atomen mit mindestens zwei Ketogruppen,
- das mindestens eine cyclische Olefin mit Z Cyclen und 7 bis 16 C-Atomen mit mindestens zwei C-C-Doppelbindungen und
- mindestens eine Verbindung mit Z-1 Cyclen und 7 bis 16 C-Atomen mit mindestens einer Aldehydgruppe,
(a2) Abtrennen des mindestens einen cyclischen Olefins mit Z Cyclen und 7 bis 16 C-Atomen mit mindestens zwei C-C-Doppelbindungen aus der Zusammensetzung (A1) aus Stufe (a1), um eine Zusammensetzung (A2) zu erhalten, mindestens enthaltend
- die mindestens eine cyclische Verbindung mit Z Cyclen und 7 bis 16 C-Atomen mit einer Ketogruppe,
- mindestens eine cyclische Verbindung mit Z Cyclen und 7 bis 16 C-Atomen mit mindestens zwei Ketogruppen und
- mindestens eine Verbindung mit Z-1 Cyclen und 7 bis 16 C-Atomen mit mindestens einer Aldehydgruppe, und
(b) destillative Behandlung der Zusammensetzung (A2) aus Schritt (a2), um eine Zusammensetzung (B) zu erhalten, enthaltend
- die mindestens eine cyclische Verbindung mit Z Cyclen und 7 bis 16 C-Atomen mit einer Ketogruppe,
- weniger als 0,5 Gew.-% der mindestens einen cyclischen Verbindung mit Z Cyclen und 7 bis 16 C-Atomen mit mindestens zwei Ketogruppen und
- weniger als 1,0 Gew.-% der mindestens einen Verbindung mit Z-1 Cyclen und 7 bis 16 C-Atomen mit mindestens einer Aldehydgruppe, wobei
sich an Stufe (b) mindestens die folgende Stufe (c) anschließt:
(c) Hydrierung der Zusammensetzung (B) in Gegenwart mindestens eines Katalysators, um eine Zusammensetzung (C) zu erhalten,
oder sich an Stufe (b) mindestens die folgende Stufe (d) anschließt:
(d) Behandeln der Zusammensetzung (B) mit wenigstens einer Base, um eine Zusammensetzung (D) zu erhalten,
und sich an Stufe (d) mindestens die folgende Stufe (c') anschließt:
(c') Hydrierung der Zusammensetzung (D) in Gegenwart mindestens eines Katalysators, um eine Zusammensetzung (C') zu erhalten, **dadurch gekennzeichnet, dass** das mindestens eine cyclische Olefin mit Z Cyclen und 7 bis 16 C-Atomen und mindestens zwei C-C-Doppelbindungen ausgewählt ist aus der Gruppe bestehend aus 1,5-Cyclooctadien, 1,5-Cyclododecadien, 1,9-Cyclohexadecadien, 1,8-Cyclotetradecadien, 1,6-Cyclododecadien, 1,6,11-Cyclopentadecatrien, 1,5,9-Cyclododecatrien, Vinylcyclohexen, Norbornadien, Ethylidennorbornen und Mischungen davon.

2. Verfahren nach Anspruch 1, **dadurch gekennzeichnet, dass** sich an Stufe (c) oder (c') mindestens die Stufe (e) anschließt:
(e) Reinigen der Zusammensetzung (C) aus Schritt (c) oder (C') aus Schritt (c'), mindestens umfassend die Schritte
(e1) thermisches Behandeln der Zusammensetzung (C) oder (C') mit mindestens einer Säure, oder mindestens einem Katalysator, der mindestens ein Übergangsmetall enthält,
(e2) weiteres Aufreinigen durch ein Verfahren ausgewählt aus der Gruppe bestehend aus Destillation, Extraktion und Kristallisation.

3. Verfahren nach einem der Ansprüche 1 oder 2, **dadurch gekennzeichnet, dass** das cyclische Olefin Cyclododecatrien ist, das durch Trimerisierung von Butadien hergestellt wurde.

4. Verfahren nach einem der Ansprüche 1 bis 3, **dadurch gekennzeichnet, dass** die Base ausgewählt ist aus der Gruppe bestehend aus Natriumhydroxid, Kaliumhydroxid und Mischungen davon.

## Claims

1. A process for preparing at least one cyclic compound with Z cycles and 7 to 16 carbon atoms with a keto group, at least comprising the stages:
(a1) oxidation of a composition (A), at least comprising one cyclic olefin with Z cycles and 7 to 16 carbon atoms and at least two C-C double bonds, by means of dinitrogen monoxide to give a composition (A1), at least comprising
- the at least one cyclic compound with Z cycles and 7 to 16 carbon atoms with a keto group,
- at least one cyclic compound with Z cycles and 7 to 16 carbon atoms with at least two keto groups,
- the at least one cyclic olefin with Z cycles and 7 to 16 carbon atoms with at least two C-C double bonds and
- at least one compound with Z-1 cycles and 7 to 16 carbon atoms with at least one aldehyde group,
(a2) separating off the at least one cyclic olefin with Z cycles and 7 to 16 carbon atoms with at least two C-C double bonds from the composition (A1) from stage (a1) in order to obtain a composition (A2), at least comprising
- the at least one cyclic compound with Z cycles and 7 to 16 carbon atoms with a keto group,
- at least one cyclic compound with Z cycles and 7 to 16 carbon atoms with at least two keto groups and
- at least one compound with Z-1 cycles and 7 to 16 carbon atoms with at least one aldehyde group, and
(b) distillative treatment of the composition (A2) from step (a2) in order to obtain a composition (B), comprising
- the at least one cyclic compound with Z cycles and 7 to 16 carbon atoms with a keto group,
- less than 0.5% by weight of the at least one cyclic compound with Z cycles and 7 to 16 carbon atoms with at least two keto groups and
- less than 1.0% by weight of the at least one compound with Z-1 cycles and 7 to 16 carbon atoms with at least one aldehyde group, where
stage (b) is followed by at least the following stage (c):
(c) hydrogenation of the composition (B) in the presence of at least one catalyst in order to obtain a composition (C),
or stage (b) is followed by at least the following stage (d):
(d) treatment of the composition (B) with at least one base in order to obtain a composition (D), and stage (d) is followed by at least the following stage (c'):
(c') hydrogenation of the composition (D) in the presence of at least one catalyst in order to obtain a composition (C'), wherein the at least one cyclic olefin with Z cycles and 7 to 16 carbon atoms and at least two C-C double bonds is selected from the group consisting of 1,5-cyclooctadiene, 1,5-cyclododecadiene, 1,9-cyclohexadecadiene, 1,8-cyclotetradecadiene, 1,6-cyclododecadiene, 1,6,11-cyclopentadecatriene, 1,5,9-cyclododecatriene, vinylcyclohexene, norbornadiene, ethylidenenorbornene and mixtures thereof.

2. The process according to claim 1, wherein stage (c) or (c') is followed by at least the stage (e):
(e) purification of the composition (C) from step (c) or (C') from step (c'), at least comprising the steps
(e1) thermal treatment of the composition (C) or (C') with at least one acid, or at least one catalyst which comprises at least one transition metal,
(e2) further purification by a process selected from the group consisting of distillation, extraction and crystallization.

3. The process according to either of claims 1 and 2, wherein the cyclic olefin is cyclododecatriene which has been prepared by trimerization of butadiene.

4. The process according to any one of claims 1 to 3, wherein the base is selected from the group consisting of sodium hydroxide, potassium hydroxide and mixtures thereof.

## Revendications

1. Procédé de fabrication d'au moins un composé cyclique contenant Z cycles et 7 à 16 atomes C contenant un groupe céto, comprenant au moins les étapes suivantes :
(a1) l'oxydation d'une composition (A), contenant au moins une oléfine cyclique contenant Z cycles et 7 à 16 atomes C et au moins deux doubles liaisons C-C, au moyen de monoxyde de diazote afin d'obtenir une composition (A1), contenant au moins
- ledit au moins un composé cyclique contenant Z cycles et 7 à 16 atomes C contenant un groupe céto,
- au moins un composé cyclique contenant Z cycles et 7 à 16 atomes C contenant au moins deux groupes céto,
- ladite au moins une oléfine cyclique contenant Z cycles et 7 à 16 atomes C contenant au moins deux doubles liaisons C-C, et
- au moins un composé contenant Z-1 cycles et 7 à 16 atomes C contenant au moins un groupe aldéhyde,
(a2) la séparation de ladite au moins une oléfine cyclique contenant Z cycles et 7 à 16 atomes C contenant au moins deux doubles liaisons C-C de la composition (A1) de l'étape (a1) afin d'obtenir une composition (A2), contenant au moins
- ledit au moins un composé cyclique contenant Z cycles et 7 à 16 atomes C contenant un groupe céto,
- au moins un composé cyclique contenant Z cycles et 7 à 16 atomes C contenant au moins deux groupes céto, et
- au moins un composé contenant Z-1 cycles et 7 à 16 atomes C contenant au moins un groupe aldéhyde, et (b) le traitement par distillation de la composition (A2) de l'étape (a2) afin d'obtenir une composition (B), contenant
- ledit au moins un composé cyclique contenant Z cycles et 7 à 16 atomes C contenant un groupe céto,
- moins de 0,5 % en poids dudit au moins un composé cyclique contenant Z cycles et 7 à 16 atomes C contenant au moins deux groupes céto, et
- moins de 1,0 % en poids dudit au moins un composé contenant Z-1 cycles et 7 à 16 atomes C contenant au moins un groupe aldéhyde,
l'étape (b) étant suivie par au moins l'étape (c) suivante :
(c) l'hydrogénation de la composition (B) en présence d'au moins un catalyseur afin d'obtenir une composition (C),
ou l'étape (b) étant suivie par au moins l'étape (d) suivante :
(d) le traitement de la composition (B) avec au moins une base afin d'obtenir une composition (D),
et l'étape (d) étant suivie par au moins l'étape (c') suivante :
(c') l'hydrogénation de la composition (D) en présence d'au moins un catalyseur afin d'obtenir une composition (C'), **caractérisé en ce que** ladite au moins une oléfine cyclique contenant Z cycles et 7 à 16 atomes C et au moins deux doubles liaisons C-C est choisie dans le groupe constitué par le 1,5-cyclooctadiène, le 1,5-cyclododécadiène, le 1,9-cyclohexadécadiène, le 1,8-cyclotétradécadiène, le 1,6-cyclododécadiène, le 1,6,11-cyclopentadécatriène, le 1,5,9-cyclododécatriène, le vinylcyclohexène, le norbornadiène, l'éthylidène-norbornène et leurs mélanges.

2. Procédé selon la revendication 1, **caractérisé en ce que** l'étape (c) ou (c') est suivie par au moins l'étape (e) :
(e) la purification de la composition (C) de l'étape (c) ou (C') de l'étape (c'), comprenant au moins les étapes suivantes :
(e1) le traitement thermique de la composition (C) ou (C') avec au moins un acide ou au moins un catalyseur qui contient au moins un métal de transition,
(e2) le traitement supplémentaire par un procédé choisi dans le groupe constitué par la distillation, l'extraction et la cristallisation.

3. Procédé selon l'une quelconque des revendications 1 ou 2, **caractérisé en ce que** l'oléfine cyclique est le cyclododécatriène, qui a été fabriqué par trimérisation de butadiène.

4. Procédé selon l'une quelconque des revendications 1 à 3, **caractérisé en ce que** la base est choisie dans le groupe constitué par l'hydroxyde de sodium, l'hydroxyde de potassium et leurs mélanges.
